# EUROPÄISCHE PATENTSCHRIFT

(11) **EP 0 986 644 B1**
(45) Veröffentlichungstag und Bekanntmachung des Hinweises auf die Patenterteilung: **04.10.2006**
(21) Anmeldenummer: 98941401.6
(22) Anmeldetag: 22.07.1998
(51) Int. Cl.: C12N 15/12, C12N 15/85, C12N 15/62, C12N 15/90, C12N 5/10, C07K 14/505, A61K 38/18

(54) **HERSTELLUNG VON ERYTHROPOIETIN DURCH ENDOGENE GENAKTIVIERUNG MIT VIRALEN PROMOTOREN**
PRODUCTION OF ERYTHROPOIETIN BY ENDOGENOUS GENE ACTIVATION WITH VIRAL PROMOTERS
PRODUCTION D'ERYTHROPOIETINE PAR ACTIVATION GENIQUE ENDOGENE AVEC DES PROMOTEURS VIRAUX

(30) Priorität: 23.07.1997 EP 97112640; 03.12.1997 DE 19753681; 10.07.1998 US 113692
(43) Veröffentlichungstag der Anmeldung: 22.03.2000
(73) Patentinhaber: Roche Diagnostics GmbH, 68305 Mannheim (DE)
(72) Erfinder: STERN, Anne, D-82377 Penzberg (DE); BRANDT, Michael, D-82393 Iffeldorf (DE); HONOLD, Konrad, D-82377 Penzberg (DE); AUER, Johannes, D-82377 Penzberg (DE); KOLL, Hans, D-82362 Weilheim (DE)
(74) Vertreter: Weiss, Wolfgang
(86) Internationale Anmeldenummer: PCT/EP1998/004590
(87) Internationale Veröffentlichungsnummer: WO 1999/005268

(56) Entgegenhaltungen:
- EP-A- 0 232 034
- EP-A- 0 267 678
- EP-A- 0 411 678
- EP-A- 0 747 485
- WO-A-91/09955
- WO-A-93/09222
- WO-A-94/12650
- WO-A-95/31560
- WO-A-96/19573
- WO-A-96/29411
- WO-A-96/35718
- RECNY M A ET AL: "STRUCTUAL CHARACTERIZATION OF NATURAL HUMAN URINARY AND RECOMBINANT DNA-DERIVED ERYTHROPOIETIN" JOURNAL OF BIOLOGICAL CHEMISTRY, Bd. 262, Nr. 35, 15. Dezember 1987, Seiten 17156-17163, XP002015827
- KRYSTAL G ET AL: "PURIFICATION OF HUMAN ERYTHROPOIETIN TO HOMOGENEITY BY A RAPID FIVE-STEP PROCEDURE" BLOOD, Bd. 67, Nr. 1, Januar 1986, Seiten 71-79, XP002052352
- SIMONSEN C C ET AL: "ISOLATION AND EXPRESSION OF AN ALTERED MOUSE DIHYDROFOLATE REDUCTASE CDNA" PROCEEDINGS OF THE NATIONAL ACADEMY OF SCIENCES OF USA, Bd. 80, Mai 1983, Seiten 2495-2499, XP002052351
- YANAGI H. ET AL: 'Recombinant Human Erythropoeitein Produced by Namalwa Cells' DNA Bd. 8, Nr. 6, 1989, MARY ANN LIEBERT INC. PUBLISHERS, Seiten 419 - 427

## Beschreibung

Die Erfindung betrifft humane Zellen, die aufgrund einer Aktivierung des endogenen humanen EPO-Gens in der Lage sind, EPO in einer ausreichenden Menge und Reinheit zu produzieren, um eine kostengünstige Herstellung von humanem EPO als pharmazeutisches Präparat zu ermöglichen. Weiterhin betrifft die Erfindung ein Verfahren zum Herstellen solcher humaner EPO-produzierender Zellen, DNA-Konstrukte zur Aktivierung des endogenen EPO-Gens in humanen Zellen sowie Verfahren zur großtechnischen Produktion von EPO in humanen Zellen.

Erythropoietin (EPO) ist ein humanes Glycoprotein, welches die Produktion von roten Blutzellen stimuliert. EPO kommt im Blutplasma von gesunden Personen nur in sehr geringen Konzentrationen vor, so daß eine Bereitstellung in größeren Mengen auf diese Weise nicht möglich ist. EP-B-0148 605 und EP-B-0205 564 beschreiben die Herstellung von rekombinantem humanen EPO in CHO-Zellen. Das in EP-B-0148 605 beschriebene EPO weist ein höheres Molekulargewicht als urinäres EPO und keine O-Glykosilierung auf. Das in EP-B-0 205 564 beschriebene EPO aus CHO Zellen ist mittlerweile in großen Mengen und in reiner Form verfügbar, es stammt jedoch aus nichthumanen Zellen. Weiterhin ist auch die Produktionsleistung von CHO Zellen oft nur relativ begrenzt.

Weiterhin ist die Gewinnung von humanem EPO aus dem Urin von Patienten mit aplastischer Anämie bekannt (Miyake et al., J. Biol. Chem. 252 (1977), 5558 - 5564). Dort wird ein siebenstufiges Verfahren offenbart, welches Ionenaustauscherchromatographie, Ethanolpräzipitation, Gelfiltration und Adsorptionschromatographie umfaßt. Dabei wird in 21%iger Ausbeute ein EPO-Präperat mit einer spezifischen Aktivität von ca. 70.000 U/mg Protein erhalten. Nachteile dieses Verfahrens und anderer Verfahren zur Gewinnung von urinärem EPO bestehen in der Beschaffung von Ausgangsmaterial in ausreichenden Mengen und mit reproduzierbarer Qualität. Weiterhin ist die Aufreinigung aus Urin schwierig und selbst ein gereinigtes Produkt ist nicht frei von urinären Verunreinigungen.

GB-A-2085 887 beschreibt ein Verfahren zur Herstellung von humanen Lymphoblastoidzellen, die in der Lage sind, EPO in geringen Mengen herzustellen. Die wirtschaftliche Produktion eines Arzneimittels mit diesen Zellen ist nicht möglich.

WO 91/06667 beschreibt ein Verfahren zur rekombinanten Herstellung von EPO. Dabei wird in einer ersten Verfahrensstufe in primären humanen embryonalen Nierenzellen das endogene EPO-Gen durch homologe Rekombination in operative Verknüpfung mit einem viralen Promotor gebracht und die DNA aus diesen Zellen isoliert. In einer zweiten Stufe wird die so isolierte DNA in nichthumane CHO-Zellen transformiert und die Expression von EPO in diesen Zellen analysiert. Es findet sich kein Hinweis, daß eine Produktion von EPO in humanen Zellen möglich ist.

WO 93/09222 beschreibt die Produktion von EPO in humanen Zellen. Dabei wird in humanen Fibroblasten, die mit einem das vollständige EPO-Gen enthaltenden Vektor transfiziert wurden, eine relativ hohe EPO Produktion von bis zu 960.620 mU/10⁶ Zellen/24 h gefunden. Diese Zellen enthalten ein exogenes EPO-Gen, das sich nicht am korrekten EPO-Genlocus befindet, so daß Probleme hinsichtlich der Stabilität der Zellinie zu erwarten sind. Angaben über eine konstitutive EPO-Produktion finden sich nicht in WO 93/092222. Darüber hinaus finden sich auch keine Angaben, ob das produzierte EPO in einer für pharmazeutische Zwecke ausreichender Qualität erhalten werden kann.

Weiterhin wird in WO 93/09222 eine Aktivierung des endogenen EPO-Gens in humanen HT1080 Zellen beschrieben. Dabei wird eine EPO Produktion von nur 2.500 mU/10⁶ Zellen/24 h (entsprechend ca. 16 ng/10⁶ Zellen/24 h) gefunden. Eine derarte geringe Produktion ist für die wirtschaftliche Produktion eines pharmazeutischen Präparats völlig ungeeignet.

WO94/12650 und WO 95/31560 beschreiben, daß eine-humane Zelle mit einem durch einen viralen Promotor aktivierten endogenen EPO-Gen in der Lage ist, nach Amplifikation des endogenen EPO-Gens EPO in einer Menge von bis zu ca. 100.000 mU/10⁶ Zellen/24 h (entsprechend ca. 0,6 µg/10⁶ Zellen/24 h) zu produzieren. Auch diese Menge reicht noch nicht für die wirtschaftliche Produktion eines Arzneimittels aus.

Die der vorliegenden Erfindung zugrundeliegende Aufgabe bestand somit darin, die oben genannten Nachteile des Standes der Technik mindestens teilweise zu beseitigen und ein technologisch besseres Verfahren zur Herstellung von EPO in humanen Zellen bereitzustellen. Insbesondere sollte hierdurch ein Produkt in einer ausreichenden Menge und Reinheit erhalten werden können, um die wirtschaftliche Herstellung für pharmazeutische Zwecke zu erlauben.

Diese Aufgabe wird durch Aktivierung des endogenen EPO Gens in humanen Zellen und gegebenenfalls durch anschließende Amplifikation des aktivierten humanen EPO Gens gelöst. Auf diese Weise ist es durch Auswahl geeigneter Ausgangszellen, DNA-Konstrukte und Selektionsstrategien überraschenderweise gelungen, humane Zellen bereitzustellen, die in der Lage sind, EPO in einer ausreichenden Menge, Qualität und Reinheit zu produzieren, die eine kostengünstige Herstellung von pharmazeutischen Präparaten erlaubt. Insbesondere nach Amplifikation des aktivierten endogenen EPO-Gens können Zellen erhalten werden, die eine deutlich höhere Produktionsleistung als die bisher zur Herstellung von rekombinantem EPO verwendeten CHO-Produktionszellen aufweisen.

Ein Gegenstand der Erfindung ist eine humane Zelle mit der Maßgabe, daß die humane Zelle keine Keimbahnzelle ist, wobei die Zelle eine Kopie eines endogenen EPO Gens in operativer Verknüpfung mit einer heterologen, in der humanen Zelle aktiven Expressionskontrollsequenz enthält und ohne vorherige Genamplifikation zur Produktion von mindestens 200 ng EPO/10⁶ Zellen/ 24 h in der Lage ist, wobei der Abstand zwischen der heterologen Expressionskontrollsequenz und dem Translationsstart des EPO-Gens nicht größer als 1100 bp ist. Vorzugsweise ist die erfindungsgemäße humane Zelle zur Produktion von 200 bis 3000 ng EPO/10⁶ Zellen/24 h und am meisten bevorzugt zur Produktion von 1000 bis 3000 ng EPO/10⁶ Zellen/24 h in der Lage.

Ein weiterer Gegenstand der vorliegenden Erfindung ist eine humane Zelle, mit der Maßgabe, daß sie keine Keimbahnzelle ist, wobei sie durch Genamplifikation aus der zuvor genannten Zelle erhältlich ist und mehrere Kopien eines endogenen EPO-Gens jeweils in operativer Verknüpfung mit einer heterologen, in der humanen Zelle aktiven Expressionskontrollsequenz enthält und zur Produktion von mindestens 1.000 ng EPO/10⁶ Zellen/24 h in der Lage ist. Besonders bevorzugt ist die durch Genamplifikation erhältliche humane Zellinie zur Produktion von 1.000 bis 25.000 ng EPO/10⁶ Zellen/24 h in der Lage und am meisten bevorzugt zur Produktion von 5.000 bis 25.000 ng EPO/10⁶ Zellen/24 h.

Die humane Zelle ist eine beliebige Zelle, mit der Maßgabe, daß sie keine Keimbahnzelle ist, vorausgesetzt daß sie in vitro kultiviert werden kann. Besonders bevorzugt sind humane Zellen, die in serumfreien Medium und insbesondere in Suspension kultivierbar sind. Auf diese Weise kann die Produktion von EPO in einem Großfermenter mit beispielsweise 1.000 1 Kulturvolumen durchgeführt werden.

Besonders bevorzugt ist die humane Zelle eine immortalisierte Zelle, beispielsweise eine HT 1080 Zelle (Rasheed et al., Cancer 33 (1974), 1027-1033), eine HeLa S3 Zelle (Puck et al., J. Exp. Med. 103 (1956), 273-284), eine Namalwa Zelle (Nadkarni et al., Cancer 23 (1969), 64-79) oder eine davon abgeleitete Zelle. Ein Beispiel für eine erfindungsgemäße Zelle ist der Klon "Aladin", der am 15.07.1997 gemäß den Vorschriften des Budapester Vertrags bei der Deutschen Sammlung von Mikroorganismen und Zellkulturen (DSMZ), Mascheroder Weg 1b, 38124 Braunschweig, unter dem Aktenzeichen DSM ACC 2320 hinterlegt wurde.

In der erfindungsgemäßen Zelle ist das endogene EPO-Gen mit einer heterologen Expressionskontrollsequenz verknüpft, die in der humanen Zelle aktiv ist. Die Expressionskontrollsequenz umfaßt einen starken viralen Promotor und vorzugsweise weitere expressionsverbessernde Sequenzen, z.B. einen Enhancer. Der Promotor kann ein regulierbarer oder ein konstitutiver Promotor sein. Vorzugsweise ist der Promotor ein SV40 - oder ein CMV Promotor. Besonders bevorzugt ist der CMV Promotor/Enhancer.

Weiterhin kann es zur Optimierung der EPO Expression bevorzugt sein, daß das endogene EPO-Gen in der humanen Zelle, das in operativer Verknüpfung mit dem heterologen Promotor ist, eine Signalpeptid-kodierende Sequenz aufweist, die von der natürlichen Signalpeptid-kodierenden Sequenz verschieden ist und vorzugsweise für ein Signalpeptid mit modifizierter Aminosäuresequenz kodiert. Besonders bevorzugt ist eine Signalpeptid-kodierende Sequenz, die für eine im Bereich der vier ersten Aminosäuren modifizierte Signalpeptidsequenz kodiert, die ausgewählt ist aus
Met-X₁-X₂-X₃
worin X₁ Gly oder Ser ist, X₂ Ala, Val, Leu, Ile, Ser oder Pro ist, X₃ und Pro, Arg, Cys oder His ist, mit der Maßgabe, daß X₁-X₂-X₃ nicht die Sequenz Gly-Val-His ist, und insbesondere aus
(a) Met-Gly-Ala-His,
(b) Met-Ser-Ala-His,
(c) Met-Gly-Val-Pro oder
(d) Met-Ser-Val-His.

Besonders bevorzugt ist die Sequenz der ersten vier Aminosäuren des Signalpeptids Met-Ser-Ala-His.

In einem weiteren Aspekt betrifft die vorliegende Erfindung ein Verfahren zum Herstellen einer humanen EPO-produzierenden Zelle, wie zuvor angegeben, umfassend die Schritte:
(a) Bereitstellen von humanen Ausgangszellen, die mindestens eine Kopie eines endogenen EPO-Gens enthalten, mit der Maßgabe, daß die humanen Ausgangszellen keine Keimbahnzellen sind,
(b) Transfizieren der Zellen mit einem DNA-Konstrukt umfassend:
   (i) zwei flankierende DNA-Sequenzen, die homolog zu Bereichen des humanen EPO Genlocus sind, um eine homologe Rekombination zu erlauben,
   (ii) ein positives Selektionsmarkergen und
   (iii) eine heterologe Expressionskontrollsequenz, die in der humanen Zelle aktiv ist, wobei die heterologe Expressionskontrollsequenz einen starken viralen Promotor umfaßt und der Abstand zwischen dem heterologen Promotor und dem Translationsstart des EPO-Gens nicht größer als 1100bp ist,
(c) Kultivieren der transfizierten Zellen unter Bedingungen, bei denen eine Selektion auf das Vorhandensein des positiven Selektionsmarkergens stattfindet,
(d) Analysieren der gemäß Schritt (c) selektierbaren Zellen und
(e) Identifizieren der EPO produzierenden Zellen.

Das zur Herstellung der humanen EPO produzierenden Zelle verwendete DNA-Konstrukt enthält zwei flankierende DNA Sequenzen, die homolog zu Bereichen des humanen EPO-Genlocus sind, um eine homologe Rekombination zu erlauben. Die Auswahl geeigneter flankierender Sequenzen erfolgt beispielsweise gemäß den in WO 90/11 354 und WO 91/09 955 beschriebenen Methoden. Vorzugsweise haben die flankierenden Sequenzen jeweils eine Länge von mindestens 150 bp. Besonders bevorzugt werden die homologen DNA-Sequenzen ausgewählt aus dem Bereich der 5'-untranslatierten Sequenzen, Exon 1 und Intron 1 des EPO Gens. Dabei ist es besonders bevorzugt, eine im Bereich von Exon 1 modifizierte DNA Sequenz, die für in den ersten Aminosäuren modifiziertes Signalpeptid kodiert, zu verwenden. Die Modifikationen im Bereich von Exon 1 sind vorzugsweise wie oben angegeben.

Das Selektionsmarkergen kann ein beliebiges für eukaryontische Zellen geeignetes Selektionsmarkergen sein, welches bei Expression zu einem selektierbaren Phänotyp führt, z. B. Antibiotikumresistenz, Auxotrophie etc. Ein besonders bevorzugtes positives Selektionsmarkergen ist das Neomycinphosphotransferasegen.

Gegebenenfalls kann auch ein negatives Selektionsmarkergen wie etwa das HSV-Thymidinkinasegen, durch dessen Expression Zellen in Gegenwart eines Selektionsmittels zerstört werden, vorhanden sein.

Wenn eine Amplifikation des in der humanen Zelle endogen aktivierten EPO-Gens erwünscht wird, enthält das DNA-Konstrukt ein Amplifikationsgen. Beispiele für geeignete Amplifikationsgene sind Dihydrofolatreduktase, Adenosindeaminase, Ornithindecarboxylase etc. Ein besonders bevorzugtes Amplifikationsgen ist das Dihydrofolatreduktasegen, insbesondere ein Dihydrofolatreduktase-Arginin-Mutante, die eine geringere Sensitivität für das selektive Agens (Methotrexat) besitzt als das Wildtyp-Gen (Simonsen et al., Proc. Natl. Acad. Sci USA 80 (1983); 2495).

Bei Anwesenheit eines Amplifikationsgens in dem zur Aktivierung des EPO Gens verwendeten DNA-Konstrukts kann das erfindungsgemäße Verfahren noch die weiteren Schritte umfassen:
(f) Amplifizieren der für EPO kodierenden DNA-Sequenz und
(g) Gewinnen von EPO-produzierenden Zellen, die eine gegenüber der Ausgangszelle erhöhte Kopienzahl einer endogenen für reifes EPO kodierenden DNA-Sequenz in operativer Verknüpfung mit einer heterologen Expressionskontrollsequenz enthalten.

Geeignete DNA-Konstrukte zur Aktivierung des in der humanen Ausgangszelle vorliegenden endogenen EPO-Gens sind die in den Beispielen aufgelisteten Plasmide p187, p189, p190 und p192. Besonders bevorzugt ist das Plasmid p189, das bei der DSMZ gemäß den Bestimmungen des Budapester Vertrags am 16.07.1997 unter dem Aktenzeichen DSM 11661 hinterlegt wurde, oder ein davon abgeleitetes Plasmid. Die DNA-Konstrukte sind vorzugsweise zirkuläre Plasmidmoleküle, die für die Transfektion der humanen Zelle in einer linearisierten Form eingesetzt werden.

Ein weiterer Gegenstand der vorliegenden Erfindung ist ein DNA-Konstrukt zur Aktivierung eines endogenen EPO-Gens in einer humanen Zelle, umfassend:
(i) zwei flankierende DNA-Sequenzen, die homolog zu Bereichen des humanen EPO-Genlocus ausgewählt aus 5'-untranslatierten Sequenzen, Exon 1 und Intron 1 sind, um eine homologe Rekombination zu erlauben, wobei im Bereich von Exon 1 eine modifizierte Sequenz vorliegt, kodierend für die Aminosäuren:
   Met-Ser-Ala-His,
(ii) ein positives Selektionsmarkergen,
(iii)eine heterologe Expressionskontrollsequenz, die in einer humanen Zelle aktiv ist, wobei die heterologe Expressionskontrollsequenz einen starken viralen Promotor umfaßt und der Abstand zwischen dem heterologen Promotor und dem Translationsstart des EPO-Gens nicht größer als 1100bp ist, und
(iv) gegebenenfalls ein Amplifikationsgen.

Noch ein weiterer Gegenstand der vorliegenden Erfindung ist ein DNA-Konstrukt zur Aktivierung eines endogenen EPO-Gens in einer humanen Zelle mit der Maßgabe, daß die humane Zelle keine Keimbahnzelle ist, wobei das Konstrukt umfaßt:
(i) zwei flankierende DNA-Sequenzen, die homolog zu Bereichen des humanen EPO-Genlocus ausgewählt aus 5'-untranslatierten Sequenzen, Exon 1 und Intron 1 sind, um eine homologe Rekombination zu erlauben,
(ii) ein positives Selektionsmarkergen,
(iii) eine heterologe Expressionskontrollsequenz, die in einer humanen Zelle aktiv ist, wobei die heterologe Expressionskontrollsequenz einen starken viralen Promotor umfaßt und der Abstand zwischen der heterologen Expressionskontrollsequenz und dem Translationstart des EPO-Gens nicht größer als 1100 bp ist, und
(iv) gegebenenfalls ein Amplifikationsgen.

Überraschenderweise wurde festgestellt, daß bei einer Modifizierung der EPO-Signalsequenz oder/und bei einer Verkürzung des Abstands zwischen der heterologen Expressionskontrollsequenz und den Translationsstart des EPO-Gens eine optimierte Expression enthalten wird. Der Abstand zwischen dem Promotor der heterologen Expressionskontrollsequenz und dem Translationsstart des EPO-Gens beträgt nicht mehr als 1100 bp, bevorzugt nicht mehr als 150 bp und stärker bevorzugt nicht mehr als 100 bp. Ein besonders bevorzugtes Beispiel für ein erfindungsgemäß zu verwendendes DNA-Konstrukt ist das Plasmid p189 (DSM 11661) oder ein davon abgeleitetes Plasmid.

Noch ein weiterer Aspekt der vorliegenden Erfindung ist ein Verfahren zur Herstellung von humanem EPO, wobei man eine erfindungsgemäße humane Zelle unter Bedingungen in einem geeignetem Medium kultiviert, bei denen eine Produktion von EPO erfolgt und das EPO aus dem Kulturmedium gewinnt. Als Medium wird vorzugsweise ein serumfreies Medium verwendet. Die Zellen werden vorzugsweise in Suspension kultiviert. Besonders bevorzugt erfolgt die Kultivierung in einem Fermenter, insbesondere in einem Großfermenter mit einem Volumen von beispielsweise 10 l - 50.000 l.

Die Gewinnung von humanem EPO aus dem Kulturmedium von humanen Zellinien umfasst vorzugsweise folgende Schritte:
(a) Leiten des Zellüberstands über ein Affinitätschromatographiemedium und Gewinnen der EPO enthaltenden Fraktionen,
(b) gegebenenfalls Leiten der EPO enthaltenden Fraktionen über ein hydrophobes Interaktionschromatographiemedium und Gewinnen der EPO enthaltenden Fraktionen,
(c) Leiten der EPO enthaltenden Fraktionen über Hydroxyapatit und Gewinnen der EPO enthaltenden Fraktionen und
(d) Aufkonzentrieren oder/und Leiten über ein Reverse Phase (RP)-HPLC-Medium.

Schritt (a) des Reinigungsverfahrens umfasst das Leiten des Zellüberstands, der gegebenenfalls vorbehandelt sein kann, über ein Affinitätschromatographiemedium. Bevorzugte Affinitätschromatographiemedien sind solche, an die ein blauer Farbstoff gekoppelt ist. Ein besonders bevorzugtes Beispiel ist Blue-Sepharose.

Nach Elution vom Affinitätschromatographiemedium wird das EPO enthaltende Eluat gegebenenfalls über ein hydrophobes Interaktionschromatograpiemedium geleitet. Dieser Schritt ist zweckmäßig, wenn ein Kulturmedium mit einem Serumgehalt ≥ 2% (v/v) verwendet wird. Bei Verwendung eines Kulturmediums mit einem geringeren Serumgehalt, z.B. 1% (v/v) oder eines serumfreien Mediums kann dieser Schritt weggelassen werden. Ein bevorzugtes hydrophobes Interaktionschromatographiemedium ist Butyl-Sepharose.

Das Eluat aus Schritt (a) oder - sofern eingesetzt - Schritt (b) wird in Schritt (c) des erfindungsgemäßen Verfahrens über Hydroxyapatit geleitet und das EPO enthaltende Eluat wird einem Aufkonzentrierungsschritt oder/und einem Reverse-Phase-HPLC-Reinigungsschritt unterzogen. Die Aufkonzentrierung erfolgt vorzugsweise durch Ausschlußchromatographie, z.B. Membranfiltration, wobei sich die Verwendung eines Mediums, z.B. einer Membran mit einer Ausschlußgröße von 10 kD als günstig erwiesen hat.

Durch das erfindungsgemäße Verfahren ist ein isoliertes humanes EPO mit einer spezifischen Aktivität von mindestens 100.000 U/mg Protein in vivo (normozythämische Maus) erhältlich, welches frei von urinären Verunreinigungen ist und sich in seiner Glykosilierung von rekombinantem EPO aus CHO Zellen unterscheiden kann. Vorzugsweise hat das erfindungsgemäße EPO eine spezifische Aktivität von mindestens 175.000, besonders bevorzugt von mindestens 200.000 bis 400.000 oder 450.000 IU/mg Protein. Das durch das erfindungsgemäße Verfahren erhältliche humane EPO kann α-2,3- oder/und α-2,6-verknüpfte Sialinsäurereste enthalten. Bei Untersuchungen von EPO aus Zellen, welche ein endogen aktiviertes EPO-Gen enthalten, wurde auf Basis der vorliegenden vorläufigen Ergebnisse das Vorhandensein von α-2,3- und α-2,6-verknüpften Sialinsäureresten gefunden. Darüber hinaus wurde auf Basis der vorliegenden vorläufigen Ergebnisse festgestellt, daß das erfindungsgemäße humane EPO einen Gehalt von weniger als 0,2% N-Glykolneuraminsäure bezogen auf den Gehalt an N-Acetylneuraminsäure aufweist.

Die Reinheit des erfindungsgemäßen humanen EPO beträgt vorzugsweise mindestens 90%, besonders bevorzugt mindestens 95% und am meisten bevorzugt mindestens 98% bezüglich des Gesamtproteingehalts. Die Bestimmung des Gesamtproteingehalts kann durch Reverse Phase HPLC, z.B. mit einer Poros R/2H-Säule erfolgen.

Weiterhin sind durch das erfindungsgemäße Verfahren humane EPO Spezies erhältlich, die sich in ihrer Aminosäuresequenz unterscheiden. So wurde beispielsweise durch massenspektrometrische Analyse (MALDI-MS) gefunden, daß aus HeLa S3 Zellen ein humanes EPO isoliert werden kann, welches überwiegend ein Polypeptid mit einer Länge von 165 Aminosäuren, das durch C-terminale Prozessierung eines Argininrests entstanden ist, und gegebenenfalls bis zu 15% eines EPO mit 166 Aminosäuren umfaßt. Weiterhin ist auch ein humanes EPO erhältlich, welches ein Polypeptid mit einer Länge von 166 Aminosäuren, d.h. ein nichtprozessiertes EPO, umfasst. Aus Namalwa Zellen konnte beispielsweise ein humanes EPO isoliert werden, welches ein Gemisch aus Polypeptiden mit einer Länge von 165 und 166 Aminosäuren umfasst.

Dieses humane EPO kann als Wirkstoff für ein pharmazeutisches Präparat eingesetzt werden, das gegebenenfalls weitere Wirkstoffe sowie pharmazeutisch übliche Hilfs-, Träger- und Zusatzstoffe enthalten kann.

In einem noch weiteren Aspekt betrifft die vorliegende Erfindung eine isolierte DNA, die für ein humanes EPO mit der im Bereich der ersten 4 Aminosäuren des Signalpeptids modifizierten Sequenz
Met-Ser-Ala-His kodiert

Die DNA kann beispielsweise eine genomische DNA oder eine cDNA sein.

Weiterhin wird die Erfindung durch die nachfolgenden Beispiele und Abbildungen und Sequenzprotokolle erläutert.

Es zeigen:
- Abbildung 1:: Eine schematische Darstellung der Amplifikation von Homologieregionen des EPO-Gens aus dem Bereich der 5'-untranslatierten Sequenzen, Exon 1 und Intron 1;
- Abbildung 2.: Eine schematische Darstellung eines Plasmids, welches EPO-Homologieregionen aus dem Bereich der 5'-untranslatierten Sequenzen, Exon 1 und Intron 1 enthält;
- Abbildung 3:: Eine schematische Darstellung einer Genaktivierungssequenz, die den Rous-Sarcomavirus-Promotor (RSV), das Neomycinphosphotransferasegen (NEO), die frühe Polyadenylierüngsregion von SV40 (SVI pA), den frühen SV40 Promotor (SVI), das Dihydrofolatreduktasegen (DHFR), eine weitere frühe SV40-Polyadenylierungsregion und den Cytomegalovirus Immediate-early-Promotor und Enhancer (MCMV) enthält;
- Abbildung 4a:: Die Herstellung des EPO-Gentargetingvektors p176;
- Abbildung 4b:: Die Herstellung der EPO-Gentargetingvektoren p179 und p187;
- Abbildung 4c:: Die Herstellung des EPO-Gentargetingvektors p189 (DSM 11661);
- Abbildung 4d:: Die Herstellung des EPO-Gentargetingvektors p190;
- Abbildung 4e:: Die Herstellung des EPO-Gentargetingvektors p192;
- Abbildung 5:: Eine schematische Darstellung der Herstellung von EPO cDNA mit Signalsequenzmutationen;

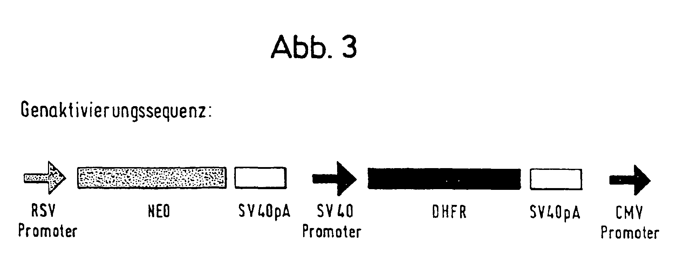

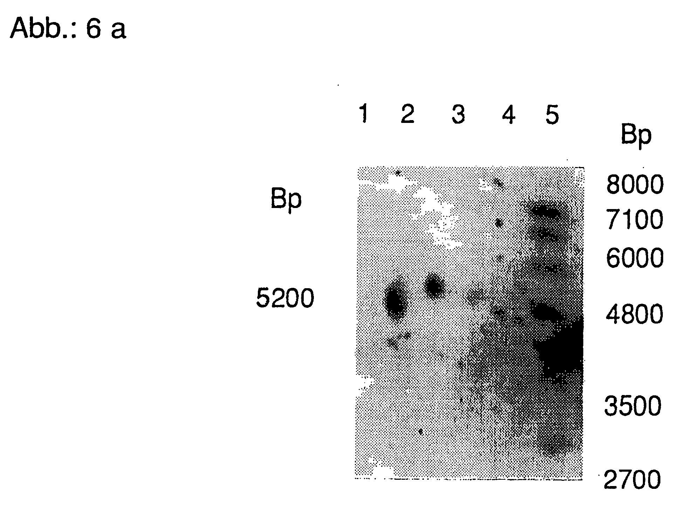

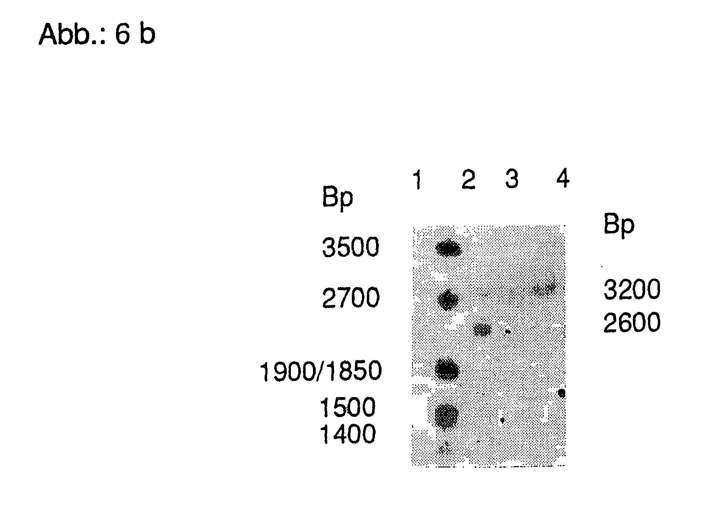

- Abbildung 6a:: Die Hybridisierung zellulärer DNA mit einer Sonde aus dem CMV-Bereich der in Abb. 3 dargestellten Gen-Kassette; die Spuren 1 bis 4 sind jeweils mit den Restriktionsenzymen AgeI und AscI gespaltene DNA aus humanen Zellen; Spur 1: EPO-produzierende HeLa S3 Zelle mit 1.000 nM MTX amplifiziert; Spur 2: EPO-produzierende HeLa S3 Zelle mit 500 nM MTX amplifiziert; Spur 3: EPO-prodzierende HeLa S3 Zelle ohne Amplifikation; Spur 4: HeLa S3 Zelle ohne aktiviertes EPO-Gen; Spur 5: Digoxigenin-markierte Längenmarker; die Größe des hybridisierenden Fragments in den Spuren 1 bis 3 ist ca. 5.200 bp und
- Abbildung 6b:: Die Hybridisierung einer Sonde aus dem kodierenden Bereich von EPO mit DNA aus humanen Zellen; Spur 1: Digoxigenin-markierte Längenmarker; Spuren 2 bis 4: DNA aus humanen Zellen gespalten mit den Restriktionsenzymen BamHI, HindIII und SalI; Spur 2: EPO produzierende HeLa S3 Zelle amplifiziert mit 500 nM MTX (Länge der durch das nichtaktivierte endogene Gen erzeugten Bande: 3.200 bp; Länge der durch Gentargeting aktivierten Kopie des EPO-Gens: 2.600 bp); Spur 3: DNA aus einer EPO produzierenden HeLa S3 Zelle nichtamplifiziert; Spur 4: DNA aus einer HeLa S3 Kontrollzelle;
- SEQ ID NO. 1 und NO. 2: Nukleotidsequenzen der zur Herstellung des PCR Produkts 1 (Abb. 1) verwendeten Primer;
- SEQ ID NO. 3 und NO. 4: Sequenzen der zur Herstellung des PCR Produkts 2 (Abb. 1) verwendeten Primer;
- SEQ ID NO. 5: Sequenz des Primers EPO EX1;
- SEQ ID NO. 6: Sequenz des Primers EX2;
- SEQ ID NO. 7: Sequenz des Primers EX3 (Met-Gly-Ala-His);
- SEQ ID NO. 8: Sequenz des vom Primer EX3 kodierten modifizierten Signalpeptidstarts;
- SEQ ID NO. 9: Sequenz des Primers EX4 (Met-Ser-Ala-His);
- SEQ ID NO. 10: Sequenz des vom Primer EX4 kodierten modifizierten Signalpeptidstarts;
- SEQ ID NO. 11: Sequenz des Primers EX5 (Met-Gly-Val-Pro);
- SEQ ID NO. 12: Sequenz des vom Primer EX5 kodierten modifizierten Signalpeptidstarts;
- SEQ ID NO. 13: Seqenz des Primers EX6 (Met-Ser-Val-His);
- SEQ ID NO. 14: Sequenz des vom Primer EX6 kodierten modifizierten Signalpeptidstarts;
- SEQ ID NO. 15: Sequenz des Primers EX Genom 1;
- SEQ ID NO. 16: Sequenz des Primers EX13 und
- SEQ ID NO. 17: Sequenz des Primers EPO EX 17.

### Beispiele

Die Aktivierung des EPO Genlocus für die Proteinproduktion im industriellen Maßstab wurde durch homologe Integration einer Genaktivierungssequenz erreicht, welche das Neomycinphosphotransferase (NEO)-Gen zur Selektion (G-418 Resistenz), das murine Dihydrofolatreduktase (DHFR)-Gen (zur Genamplifikation durch MTX) und den Cytomegalovirus (CMV)-Immediate-Early-Promotor und Enhancer zur Genaktivierung enthält.

### Beispiel 1 Klonierung von EPO-Homologieregionen

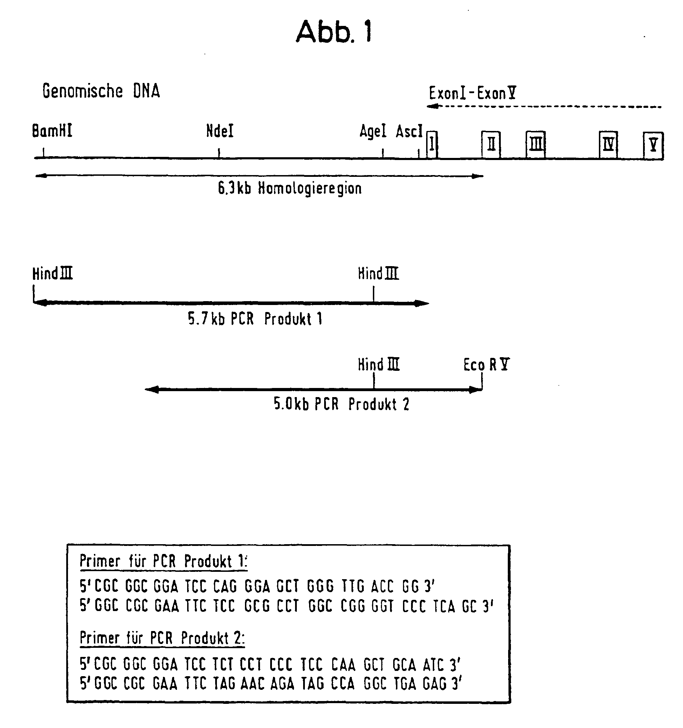

Homologieregionen des EPO-Gens wurden durch PCR unter Verwendung einer genomischen Placenta DNA (Boehringer Mannheim) amplifiziert. Dabei wurden aus einer 6,3 kB langen Homologieregion aus dem Bereich der 5'-untranslatierten Sequenzen des EPO-Gens, Exon 1 und Intron 1 zwei PCR-Produkte hergestellt (vgl. Figur 1). Die zur Herstellung des PCR-Produkts 1 verwendeten Primer hatten folgende Sequenzen: 5'-CGC GGC GGA TCC CAG GGA GCT GGG TTG ACC GG-3' (SEQ ID NO. 1) und 5'-GGC CGC GAA TTC TCC GCG CCT GGC CGG GGT CCC TCA GC-3' (SEQ ID NO. 2). Die zur Herstellung des PCR-Produkts 2 verwendeten Primer hatten folgende Sequenzen: 5'-CGC GGC GGA TCC TCT CCT CCC TCC CAA GCT GCA ATC-3' (SEQ ID NO. 3) und 5'-GGC CGC GAA TTC TAG AAC AGA TAG CCA GGC TGA GAG-3' (SEQ ID NO. 4).

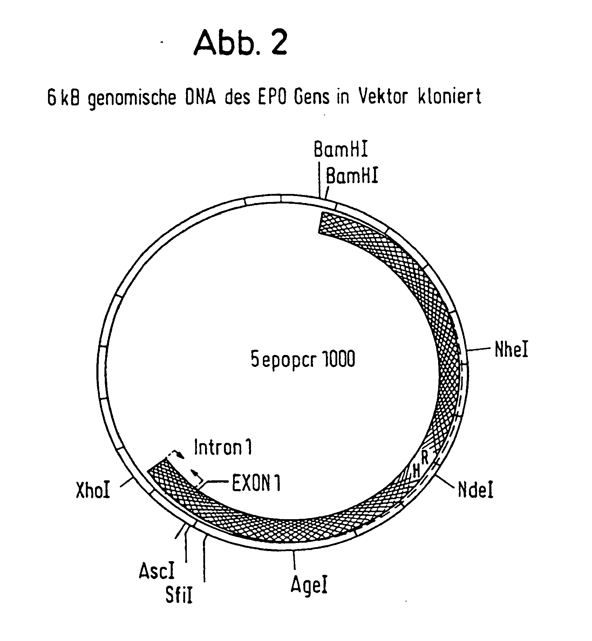

Aus den PCR-Produkten 1 und 2 wurden die gewünschten Segmente durch Restriktionsspaltung (PCR-Produkt 1: HindIII, PCR-Produkt 2: HindIII und Eco RV) herausgeschnitten und in den Vektor pCRII (Invitrogen), der mit Hind III und Eco RV gespalten war, kloniert. Der auf diese Weise erhaltene rekombinante Vektor wurde Sepopcr1000 bezeichnet (vgl. Figur 2).

### Beispiel 2 Konstruktion von EPO Gen Targetingvektoren

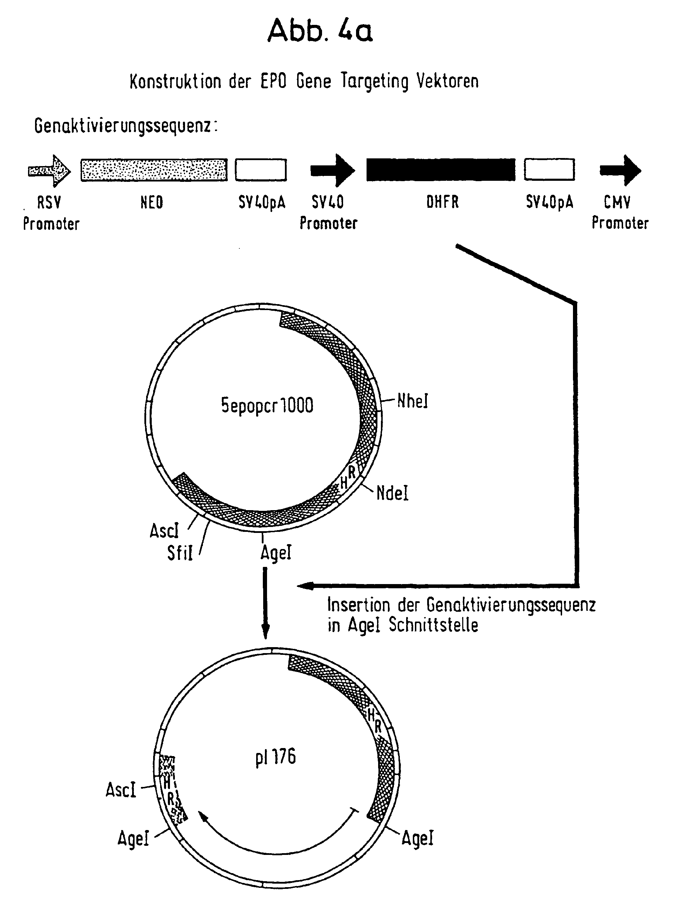

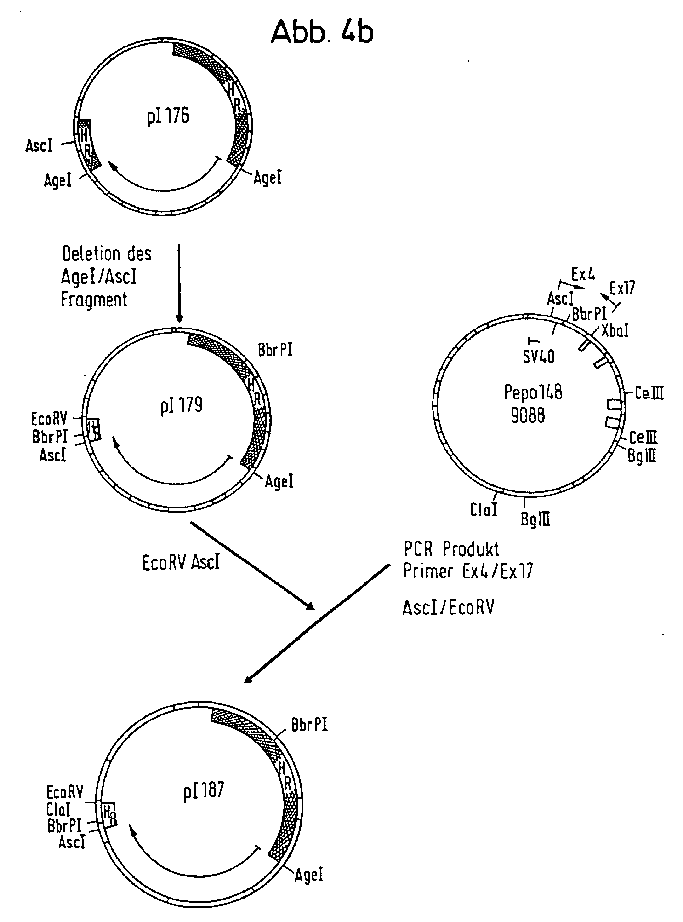

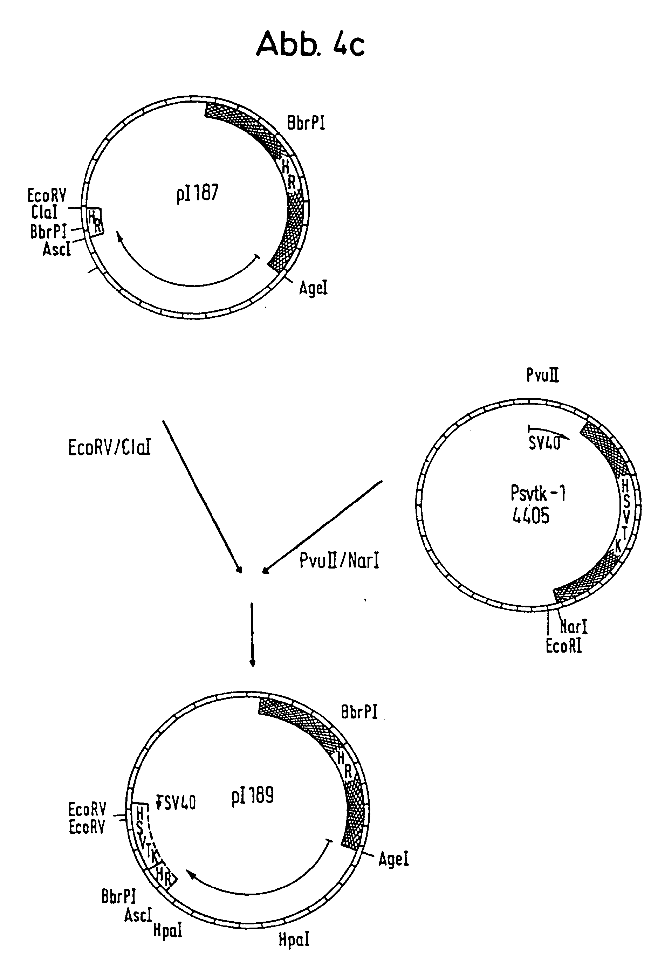

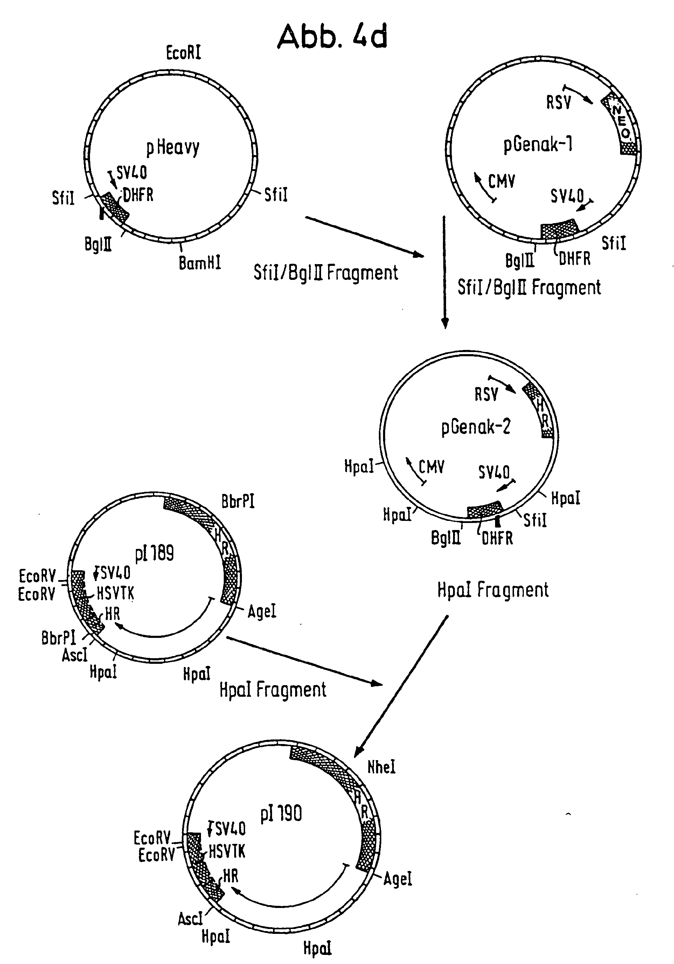

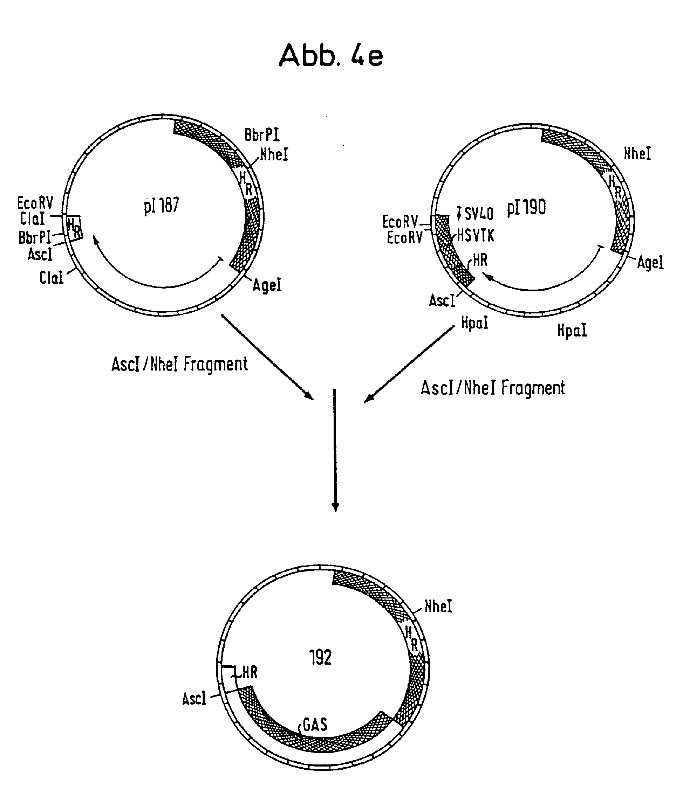

2.1 Eine Genaktivierungssequenz, die das NEO-Gen, das DHFR Gen und einen CMV Promotor/Enhancer enthält (vgl. Fig. 3) wurde in die AgeI Stelle des die EPO Homologieregion enthaltenden Plasmids 5epocr1000 inseriert, wobei das Plasmid p176 erhalten wurde (vgl. FIG. 4a). Um den CMV Promotor möglichst nahe an die Translationstartstelle des EPO Gens zu bringen, wurde ein 963 bp langes Segment zwischen den Restriktionsstellen AscI und AgeI (partielle Spaltung) deletiert, wobei das Plasmid p179 erhalten wurde (Fig. 4b).
2.2 Um eine Optimierung der Expression zu erreichen, wurden Nukleotide in Exon 1, die für den Beginn der EPO-Leadersequenz Met-Gly-Val-His kodieren, durch die synthetische Sequenz Met-Ser-Ala-His ersetzt (vgl. auch Beispiel 6). Diese Sequenz wurde durch Amplifikation einer genomischen EPO-DNA-Sequenz, z.B. des Plasmids pEPO148, das ein 3,5 kB BstEII/EcoRI-Fragment (inklusive der Exons 1-5) der humanen EPO Gensequenz unter Kontrolle des SV40 Promotors enthält (Jacobs et al., Nature 313 (1985), 806 und Lee-Huang et al., Gene 128 (1993), 227), als Matrize mit den Primern Ex4 (SEQ ID NO. 9) und Ex17 (SEQ ID NO. 17) (Tabelle 1) erhalten. Dabei wurde das Plasmid p187 erhalten (Fig. 4b).
2.3 Das Plasmid p189 wurde aus dem Plasmid p187 durch Insertion des Herpes Simplex Virus-Thymidinkinasegens (HSV-TK), welches aus Psvtk-1 (PvuII/NarI Fragment) stammte, hergestellt (Fig. 4c). Das HSV-TK-Gen befindet sich unter Kontrolle des SV40 Promotors am 3'-Ende von Intron 1 (Eco RV/ClaI) in entgegengesetzter Orientierung relativ zum CMV Promotor und sollte zur negativen Selektion auf eine homologe Rekombination dienen.
2.4 Zur Konstruktion von Plasmid p190 wurde ein SfiI/BglII Fragment von pHEAVY, einem Plasmid, das die cDNA einer bei Simonsen et al. (Proc. Natl. Acad. Sci. USA 80 (1983), 2495) beschriebene Arginin-Mutante von DHFR enthält, in das mit SfiI und BglII geschnittene Plasmid pGenak-1 subkloniert, das das NEO-Gen unter Kontrolle des RSV-Promotors und der späten SV40 Polyadenylierungsstelle als Terminator, das murine DHFR-Gen unter Kontrolle des frühen SV40 Promotors und der frühen SV40 Polyadenylierungsstelle als Terminator (Kaufmann et al., Mol. Cell. Biol. 2 (1982), 1304; Okayama et al., Mol. Cell. Biol. 3 (1983), 280 und Schimke, J. Biol. Chem. 263 (1988), 5989) und den CMV-Promotor (Boshart et al., Cell 41 (1995), 521) enthält. Anschließend wurde ein HpaI Fragment, welches die für die DHFR-Arginin-Mutante kodierende cDNA enthielt, in das mit HpaI geschnittene Plasmid p189 ligiert, wobei das Plasmid p190 erhalten wurde (Fig. 4d).
2.5 Um einen Transfektionsvektor ohne das HSV-TK Gen zu erhalten, wurde ein AscI/NheI Fragment des Plasmid p190, welches die Genaktivierungssequenz enthielt, in ein das Exon 1 enthaltendes AscI/NheI Fragment des Plasmids p187 ligiert. Das resultierende Plasmid wurde p192 genannt (Fig. 4e).

### Beispiel 3 Transfektion von Zellen

Es wurden verschiedene Zellinien auf die Produktion von EPO selektioniert und mit Targetingvektoren transfiziert.

### 3.1 Namalwazellen

Die Zellen wurden in T150 Gewebekulturflaschen gezüchtet und durch Elektroporation (1 x 10⁷ Zellen/800 µl Elektroporationspuffer 20 mM Hepes, 138 mM NaCl, 5 mM KCl, 0,7 mM Na₂HPO₄, 6 mM D-Glucose-Monohydrat pH 7,0, 10 µg linearisierte DNA, 960 µF, 260 V BioRad Gene Pulser) transfiziert. Nach der Elektroporation wurden die Zellen in RPMI 1640, 10% (v/v) fötalem Kälberserum (FCS), 2 mM L-Glutamin, 1 mM Natriumpyruvat in vierzig 96-Lochplatten gezüchtet. Nach zwei Tagen wurden die Zellen für 10 bis 20 Tage in 1 mg/ml G-418 enthaltendem Medium kultiviert. Der Überstand wurde in einem Festphasen-ELISA auf die Produktion von EPO getestet (siehe Beispiel 4). Die EPO produzierenden Klone wurden in 24-Lochplatten und T-25 Gewebekulturflaschen expandiert. Aliquots wurden eingefroren und die Zellen wurden durch FACS (Ventage, Beckton Dickinson) subkloniert. Die Subklone wurden wiederholt auf EPO-Produktion gestestet.

### 3.2 HT 1080 Zellen

Die Bedingungen waren wie für Namalwazellen beschrieben, außer daß die HT1080 Zellen in DMEM, 10% (v/v) FCS, 2 mM L-Glutamin, 1 mM Natriumpyruvat kultiviert wurden. Zur Transfektion durch Elektroporation wurden die Zellen durch Typsinisierung von den Wänden der Kulturgefäße abgelöst. Nach der Elektroporation wurden 1 x 10⁷ Zellen in DMEM, 10% (v/v) FCS, 2 mM L-Glutamin, 1 mM Natriumpyruvat in 5 96-Lochplatten kultiviert.

### 3.3 HeLa S3 Zellen

Die Bedingungen waren wie für Namalwalzellen beschrieben, außer daß die Helazellen in RPM 1640, 10% (v/v) FCS, 2 mM L-Glutamin, 1% (v/v) MEM nichtessentiellen Aminosäuren (Sigma), 1 mM Natriumpyruvat kultiviert wurden. Zur Transfektion durch Elektroporation wurden die Zellen durch Trypsinisierung von den Wänden der Kulturgefäße abgelöst. Die Bedingungen für die Elektroporation waren 960 µF/250 V. Nach der Elektroporation wurden die Zellen in RPMI 1640, 10% (v/v) FCS, 2 mM L-Glutamin, 1% (v/v) MEM, 1 mM Natriumpyruvat in T75 Gewebekulturflaschen kultiviert. 24 Stunden nach Elektroporation wurden die Zellen trypsinisiert und für 10 bis 15 Tage in einem 600 µg/ml G-418 enthaltenden Medium in 10 96-Lochplatten kultiviert.

### Beispiel 4 Selektion auf EPO produzierende Klone

Der Kulturüberstand von transfizierten Zellen wurde in einem EPO ELISA getestet. Alle Schritte wurden bei Raumtemperatur durchgeführt. Mit Streptavidin vorbeschichtete 96-Lochplatten wurden mit biotinylierten Anti-EPO-Antikörpern (Boehringer Mannheim) beschichtet. Zur Beschichtung wurden die Platten zunächst mit 50 mM Natriumphosphat pH 7,2, 0,05% (v/v) Tween 20 gewaschen. Dann wurden pro Loch 0,01 ml Beschichtungspuffer (4 µg/ml biotinylierter Antikörper, 10 mM Natriumphosphat pH 7,2, 3 g/l Rinderserumalbumin, 20 g/l Saccharose, 9 g/l NaCl) zugegeben und 3 h bei Raumtemperatur inkubiert. Dann wurden die Platten mit 50 mM Natriumphosphat pH 7,2 gewaschen, getrocknet und eingeschweißt.

Vor dem Test wurden die Platten nach dreimaligem Waschen mit 0,3 ml phosphatgepufferter Salzlösung (PBS), 0,05 % Tween 20 (Sigma) über Nacht mit 0,2 ml PBS, 1 % (w/v) Crotein (Boehringer Mannheim) pro Loch inkubiert, um unspezifische Bindungen zu blockieren.

Nach Entfernung der Blockierungslösung wurden 0,1 ml Kulturüberstand zugegeben und die Platten wurden über Nacht inkubiert. Die einzelnen Löcher wurden dreimal mit jeweils 0,3 ml PBS, 0,05 % Tween 20 gewaschen. Dann wurden 100 µl Peroxidase (POD)-konjugierter monoklonaler Antikörper (Boehringer Mannheim, 150 mU/ml) für zwei Stunden zugegeben. Die Löcher wurden anschließend wieder dreimal mit jeweils 0,3 ml PBS, 0,05 % Tween 20 gewaschen. Dann wurde die Peroxidasereaktion unter Verwendung von ABTS® als Substrat in einem Perkin Elmer Photometer bei 405 nm durchgeführt. Eine Standardeichkurve unter Verwendung von rekombinantem EPO aus CHO-Zellen (Boehringer Mannheim, 100-1000 pg/Loch) wurde zur Berechnung der EPO-Konzentrationen verwendet.

### Beispiel 5 EPO-Genamplifizierung

Zur Erhöhung der EPO Expression wurden die EPO produzierenden Klone in Gegenwart steigender Konzentrationen (100 pM-1000 nM) Methotrexat (MTX) kultiviert. Bei jeder MTX Konzentration wurden die Klone durch einen ELISA (siehe Beispiel 4) auf Produktion von EPO getestet. Starke Produzenten wurden durch limitierte Verdünnung subkloniert.

### Beispiel 6 Signalsequenzmutationen

Um die Leadersequenz des EPO Moleküls zu optimieren, wurden die ersten von Exon 1 kodierten Aminosäuren ausgetauscht.

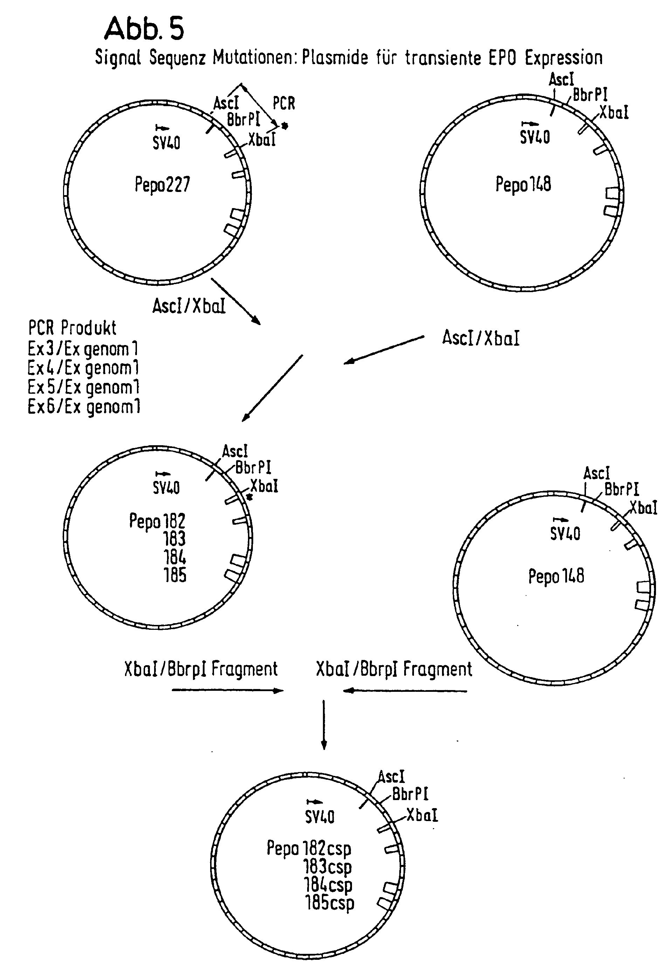

Primer mit verschiedenen Sequenzen (SEQ ID NO. 4-17; der 3'-Primer enthielt eine CelII Stelle zur Selektion von modifizierten Sequenzen) wurden verwendet, um ein AscI/XbaI Fragment durch PCR unter Verwendung von Plasmid pEPO227, das ein 4 kB HindIII/EcoRI Fragment (inklusive der Exon 1-5) der humanen EPO-Gensequenz unter Kontrolle des SV40 Promotors enthält (Jacobs et al., Nature 313 (1985), 806; Lee-Huang et al., Gene 128 (1993), 227), als Matritze zu erhalten. Die resultierenden Fragmente wurden anschließend in das Plasmid pEPO148 (Beispiel 2.2) kloniert, wobei die Plasmide pEPO 182, 183, 184 und 185 erhalten wurden (Figur 5). Die EPO-Genexpression wurde durch einen SV40 Promotor angetrieben. COS-7 Zellen wurden transient mit den Konstrukten (DEAE-Dextranmethode) transfiziert und die Zellen wurden 48 h nach der Transfektion auf die EPO Produktion getestet.

Die auf diese Weise erhaltene mutierte Leadersequenz Met-Ser-Ala-His mit der besten EPO-Expression wurde zur Konstruktion der Gentargetingvektoren eingesetzt (vgl. Beispiel 2.2).

### Beispiel 7 Charakterisierung von EPO-produzierenden Zellinien

Drei verschiedene Zellinien (Namalwa, HeLa S3 und HT 1080) wurden zur EPO Genaktivierung ausgewählt. EPO-produzierende Klone wurden durch Transfektion mit den Plasmiden p179, p187, p189, p190 oder p192 erhalten (vgl. Beispiele 2 und 3).

Es wurden ca. 160.000 NEO-resistente Klone auf die EPO-Produktion getestet, von denen 12-15 EPO in signifikanter Ausbeute reproduzierbar in den Zellüberstand sekretierten.

Davon konnten überraschenderweise ohne Genamplifikation durch MTX insgesamt 7 EPO-Klone identifiziert werden, die EPO in ausreichenden Mengen für ein großtechnische Produktion erzeugten. Die EPO-Produktion dieser Klone lag im Bereich von 200 ng/ml bis mehr als 1000 ng/ml/10⁶ Zellen/24 h. Ein Beispiel einer solchen Zelle ist der bei der DSMZ hinterlegte Klon "Aladin" (DSM ACC 2320), der aus einer Namalwazelle erhalten wurde.

Nach Genamplifikation mit 500 nM MTX konnte die EPO-Produktion der identifizierten EPO-Klone auf bis zu mehr als 3000 ng/ml/10⁶ Zellen/24 h gesteigert werden. Eine weitere Erhöhung der MTX Konzentration auf 1000 nM führte zu einer Produktion von bis zu mehr als 7000 ng/ml/10⁶ Zellen/24 h.

Die erhaltenen Klone zeigten eine EPO Produktion auch unter serumfreien Kulturbedingungen.

### Beispiel 8 Charakterisierung des Genoms vom EPO-produzierenden Klonen

### 8.1 Methodik

Humane genomische DNA wurde aus ca. 10⁸ Zellen isoliert und quantifiziert (Sambrook et al., 1989). Nach Spaltung der genomischen DNA mit Restriktionsenzymen, z. B. AgeI und AscI bzw. BamHI, Hind III und SalI wurden die DNA Fragmente durch Agarosegelelektrophorese ihrer Größe nach aufgetrennt und schließlich auf eine Nylonmembran transferiert und immobilisiert.

Die immobilisierte DNA wurde mit Digoxigenin-markierten EPO- oder Genaktivierungssequenz-spezifischen DNA-Sonden hybridisiert (DIG DNA Labeling Kit, Boehringer Mannheim) und unter stringenten Bedingungen gewaschen. Die spezifischen Hybridisierungssignale wurden mit Hilfe eine Chemilumineszenzverfahrens unter Einsatz strahlungsempfindlicher Filme detektiert.

### 8.2 Ergebnisse

Die Behandlung von Zellen mit 500 nM MTX führte zu einer Verstärkung des Hybridisierungssignals im EPO-Locus um den Faktor 5 bis 10. Bei einer weiteren Steigerung auf 1000 nM MTX wurde eine Verstärkung um den Faktor > 10 erhalten (Figur 6a).

Im Falle der Hybridisierung mit der EPO-spezifischen Sonde wurden auch die durch homologe Rekombination nicht getroffenen Kopien des Chromosoms 7 detektiert. Wie in Figur 6b zu sehen ist, weisen diese ebenfalls hybridisierenden DNA-Fragmente eine andere, deutlich unterscheidbare Größe auf und werden durch den Einsatz von MTX nicht in ihrer Signalstärke verändert.

### Beispiel 9 Aufreinigung von EPO aus Kulturüberständen humaner Zellinien (Hela S3; Namalwa und HT1080)

Im wesentlichen wurden zur Aufreinigung von EPO aus Zellkulturüberständen humaner Zellinien zwei Methoden verwendet, die sich in Anzahl und Prinzip der Chromatographieschritte unterschieden und abhängig von der Zusammensetzung des Mediums und der EPO-Konzentration eingesetzt wurden:

| | | |
|---|---|---|
| Methode 1: | 1. Schritt: | Blue-Sepharose-Säule |
| | 2. Schritt: | Butyl-Sepharose-Säule |
| | 3. Schritt: | Hydroxyapatit-Säule |
| | 4. Schritt: | Aufkonzentrierung |
| Methode 2: | 1. Schritt: | Blue-Sepharose-Säule |
| | 2. Schritt: | Hydroxyapatit-Säule |
| | 3. Schritt: | Aufkonzentrierung |
| | (alternativer 3. Schritt: | RP-HPLC) |

Beispiel für eine Aufreinigung eines HeLaS3-Zellkulturüberstandes mit 2 % (v/v) fötalem Kälberserum (FKS) nach Methode 1:

### 1. Blue Sepharose-Säule:

Eine 5 ml Hi-Trap-Blue-Säule (Blue-Sepharose-Fertigsäule von Pharmacia) wurde mit mindestens 5 Säulenvolumina (SV) Puffer A (20 mM Tris-HCl, pH, 7,0; 5 mM CaCl₂; 100 mM NaCl) äquilibriert. Anschließend wurden 70 ml Hela-Zellüberstand (ca. 245 µg EPO und 70-100 mg Gesamtprotein enthaltend) über Nacht bei einem Fluß von 0,5 ml/min im Kreislaufverfahren aufgezogen.

Die Säule wurde mit mindestens 5 SV Puffer B (20mM Tris-HCl, pH 7,0; 5 mM CaCl₂; 250 mM NaCl) und mindestens 5 SV Puffer C (20mM Tris-HCl, pH 7,0; 0,2 mM CaCl₂, 250 mM NaCl) bei 0,5 ml/min gewaschen. Der Wascherfolg wurde über die Messung des Proteingehalts bei OD280 verfolgt.

Die Elution von EPO erfolgte mit Puffer D (100 mM Tris-HCl, pH 7,0; 0,2 mM CaCl₂; 2 M NaCl) bei einem Fluß von 0,5 ml/min. Die Elutionslösung wurde in 1-2 ml Fraktionen gesammelt.

Der EPO-Gehalt der Fraktionen, der Waschlösungen und des Durchlaufs wurden über Reverse-Phase (RP)-HPLC durch Auftrag eines Aliquots auf eine POROS R2/H-Säule (Boehringer Mannheim) bestimmt. Alternativ wurde zur qualitativen Identifizierung EPO-haltiger Fraktionen ein immunologischer Dotblot durchgeführt.

EPO-haltige Fraktionen (8-12 ml) wurden gepoolt und auf eine Butyl-Sepharose-Säule aufgetragen.

Die Ausbeute nach der Blue-Sepharose-Säule betrug ca. 175 µg EPO (entspricht ca. 70%). Im Allgemeinen betrug die Ausbeute nach Blue-Sepharose zwischen 50-75%.

### 2. Butyl-Sepharose-Säule (Hydrophobe-Interaktions-Chromatographie)

Eine selbsthergestellte 2-3 ml Butyl-Sepharose-Säule (Material: Toyopearl Butyl S650) wurde mit mindestens 5 SV Puffer D (100 mM Tris-HCl, pH 7,0; 0,2 mM CaCl₂; 2 M NaCl) äquilibriert und anschließend der EPO-haltige Blue-Sepharose-Pool aus 1. (ca. 150 µg EPO) bei einem Fluß von 0,5 ml/min aufgezogen.

Die Säule wurde mit mindestens 5 SV Puffer E (20 mM Tris-HCl, pH 7,0; 2 M NaCl und 10 % Isopropanol) bei 0,5 ml/min gewaschen. Der Wascherfolg wurde über die Messung des Proteingehalts bei OD280 verfolgt.

Die Elution von EPO erfolgte mit Puffer F (20 mM Tris-HCl, pH 7,0; 2 M NaCl und 20 % Isopropanol) bei einem Fluß von 0,5 ml/min. Die Elutionslösung wurde in 1-2 ml Fraktionen gesammelt.

Der EPO-Gehalt der Fraktionen, der Waschlösungen und des Durchlaufs wurden über RP-HPLC durch Auftrag eines Aliquots auf eine POROS R2/H-Säule bestimmt. Alternativ wurde zur qualitativen Identifizierung EPO-haltiger Fraktionen ein immunologischer Dotblot durchgeführt.

EPO-haltige Fraktionen (10-15 ml) wurden gepoolt und auf eine Hydroxyapatit-Säule aufgetragen.

Die Ausbeute der Butyl-Sepharose-Säule betrug ca. 130 µg EPO (entspricht ca. 85 %). Im Allgemeinen betrug die Ausbeute der Butyl-Sepharose zwischen 60-85% vom Auftrag der Blue-Sepharose-Pools.

### 3. Hydroxyapatit-Säule

Eine 5 ml Hydroxyapatit-Säule (Econo-Pac CHT II-Fertigsäule von BioRAD) wurde mit mindestens 5 SV Puffer F (20 mM Tris-HCl, pH 7,0; 2 M NaCl; 20% Isopropanol) äquilibriert und anschließend der EPO-haltige Butyl-Sepharose-Pool aus 2. (ca. 125 µg EPO) bei einem Fluß von 0,5 ml/min aufgezogen.

Die Säule wurde mit mindestens 5 SV Puffer G (20 mM Tris-HCl, pH 7,0; 2 M NaCl) bei 0,5 ml/min gewaschen. Der Wascherfolg wurde über die Messung des Proteingehalts bei OD280 verfolgt.

Die Elution von EPO erfolgte mit Puffer H (10 mM Na-Phosphat, pH 7,0; 80 mM NaCl) bei einem Fluß von 0,5 ml/min. Die Elutionslösung wurde in 1-2 ml Fraktionen gesammelt.

Der EPO-Gehalt der Fraktionen, der Waschlösungen und des Durchlaufs wurden über RP-HPLC durch Auftrag eines Aliquots auf eine POROS R2/H-Säule bestimmt.

EPO-haltige Fraktionen (3-6 ml) wurden gepoolt. Die Ausbeute der Hydroxyapatit-Säule betrug ca. 80 µg EPO (entspricht ca. 60%). Im Allgemeinen betrug die Ausbeute der Hydroxyapatit-Säule zwischen 50-65% vom Auftrag der Butyl-Sepharose-Pools.

### 4. Aufkonzentrierung

Die gepoolten EPO-Fraktionen aus dem Hydroxyapatit-Schritt wurden in Zentrifugationseinheiten mit einer Ausschlußgröße von 10 kD (z.B. Microsep von Filtron) auf eine Konzentration von 0,1-0,5 mg/ml aufkonzentriert, mit 0,01% Tween 20 versetzt und in Aliquots bei -20°C gelagert.

### Ausbeuteschema:

| | EPO (µg) | Ausbeute (%) |
|---|---|---|
| Ausgang | 245 | 100 |
| Blue-Sepharose | 175 | 70 |
| Butyl-Sepharose-Säule | 130 | 53 |
| Hydroxyapatit-Säule | 80 | 33 |
| Aufkonzentrierung | 60 | 25 |

Die Reinheit des isolierten EPO war etwa > 90%, in der Regel sogar > 95%.

Zur Erhöhung der EPO-Ausbeute wurde auch die Methode 2 verwendet, bei der der Butyl-Sepharose-Schritt fehlte. Vor allem bei Zellkulturüberständen ohne oder mit 1% (v/v) FKS-Zusatz ist diese Methode anwendbar und liefert isoliertes EPO von ungefähr gleicher Reinheit (90-95%). Die Anwesenheit von 5 mM CaCl₂ im Äquilibrierungspuffer (Puffer F) für die Hydroxyapatit-Säule führte bei dieser Methode zu einer verbesserten Bindung und damit auch zu einem reproduzierbaren Elutionsverhalten von EPO beim Hydroxyapatit-Schritt. Deshalb wurde bei Methode 2 bei prinzipiell gleichem Ablauf wie Methode 1 mit folgenden Puffern durchgeführt:

### 1. Blue-Sepharose-Säule:

| | |
|---|---|
| Äquilibrierpuffer (Puffer A): | 20 mM Tris-HCl, pH 7,0; 5 mM CaCl₂; 100 mM NaCl |
| Waschpuffer 1 (Puffer B): | 20 mM Tris-HCl, pH 7,0; 5 mM CaCl₂; 250 mM NaCl |
| Waschpuffer 2 (Puffer C): | 20 mM Tris-HCl, pH 7,0; 5 mM CaCl₂, 250 mM NaCl |
| Elutionspuffer (Puffer D): | 100 mM Tris-HCl, pH 7,0; 5 mM CaCl₂; 2 M NaCl |

### 2. Hydroxvapatit-Säule

| | |
|---|---|
| Äquilibrierpuffer (Puffer F): | 50 mM Tris-HCl, pH 7,0; 5 mM CaCl₂; 1 M NaCl |
| Waschpuffer (Puffer G) : | 10 mM Tris-HCl, pH 7,0; 5 mM CaCl₂; 80 mM NaCl |
| Elutionspuffer (Puffer H): | 10 mM Na-Phosphat, pH 7,0; 0,5 mM CaCl₂; 80 mM NaCl |

### Ausbeuteschema:

| | EPO (µg) | Ausbeute (%) |
|---|---|---|
| Ausgang | 600 | 100 |
| Blue-Sepharose | 450 | 75 |
| Hydroxyapatit-Säule | 335 | 55 |
| Aufkonzentrierung | 310 | 52 |

Der Zusatz von 5 mM CaCl₂ in die Puffer B bis G bei Methode 1 führte ebenfalls zu einer besseren Bindung und definierteren Elution von der Hydroxyapatit-Säule.

### Beispiel 10 Bestimmung der spezifischen Aktivität in vivo von EPO aus humanen Zellinien (Bioassay an der normozythämischen Maus)

Die dosis-abhängige Aktivität von EPO auf die Vermehrung und Differenzierung von Erythrocyten-Vorläuferzellen wurde in vivo in Mäusen über den Anstieg der Reticulocyten im Blut nach EPO-Gabe bestimmt.

Hierzu wurde je acht Mäusen verschiedene Dosen der zu analysierenden EPO-Probe und eines EPO-Standards (abgeglichen gegen den EPO-WHO-Standard) parenteral verabreicht. Die Mäuse wurden anschließend unter konstanten, definierten Bedingungen gehalten. 4 Tage nach EPO-Gabe wurden den Mäusen Blut entnommen und die Reticulocyten mit Acridinorange angefärbt. Die Bestimmung der Reticulocytenzahl pro 30000 Erythrocyten erfolgte mikrofluorimetrisch im Durchflußcytometer durch Analyse des Rotfluoreszenz-Histogramms.

Die Berechnung der biologischen Aktivität erfolgte aus den Werten für die Reticulocytenzahlen der Probe und des Standards bei den unterschiedlichen Dosen nach dem von Linder beschriebenen Verfahren der paarweisen Gehaltsbestimmung mit parallelen Geraden (A. Linder, Planen und Auswerten von Versuchen, 3. Auflage, 1969, Birkenhäuser Verlag Basel).

### Ergebnis:

| EPO aus Zellinie | spezifische Aktivität U/mg |
|---|---|
| Hela S3 (Probe 1) | 100.000 |
| Hela S3 (Probe 2) | 110.000 |

## Patentansprüche

1. Humane Zelle,
**dadurch gekennzeichnet,**
**dass** sie eine Kopie eines endogenen EPO-Gens in operativer Verknüpfung mit einem heterologen, in der humanen Zelle aktiven Promotor enthält und zur Produktion von mindestens 200 ng EPO/10⁶ Zellen/24 h in der Lage ist, wobei der Abstand zwischen dem heterologen Promotor und dem Translationsstart des EPO-Gens nicht größer als 1100 bp ist, erhältlich durch ein Verfahren umfassend die Schritte:
(a) Bereitstellen von humanen Ausgangszellen, die mindestens eine Kopie eines endogenen EPO-Gens enthalten, mit der Maßgabe, dass die humanen Ausgangszellen keine Keimbahnzellen sind,
(b) Transfizieren der Zellen mit einem DNA-Konstrukt umfassend:
(i) zwei flankierende DNA-Sequenzen, die homolog zu Bereichen des humanen EPO-Genlocus sind, um eine homologe Rekombination zu erlauben,
(ii) ein positives Selektionsmarkergen und
(iii) eine heterologe Expressionskontrollsequenz, die in der humanen Zelle aktiv ist, wobei die heterologe Expressionskontrollsequenz einen starken viralen Promotor umfasst und der Abstand zwischen dem heterologen Promotor und dem Translationsstart des EPO-Gens nicht größer als 1100 bp ist,
(c) Kultivieren der transfizierten Zellen unter Bedingungen, bei denen eine Selektion auf das Vorhandensein des positiven Selektionsmarkergens stattfindet,
(d) Analysieren der gemäß Schritt (c) selektierbaren Zellen und
(e) Identifizieren der EPO-produzierenden Zellen.

2. Humane Zelle nach Anspruch 1,
**dadurch gekennzeichnet,**
**dass** sie zur Produktion von 200 - 3000 ng EPO/10⁶ Zellen/24 h in der Lage ist.

3. Humane Zelle nach Anspruch 1 oder 2,
**dadurch gekennzeichnet,**
**dass** sie die Zelle DSM ACC 2320 ist.

4. Humane Zelle, erhältlich durch Genamplifikation aus einer Zelle nach einem der Ansprüche 1 bis 3,
**dadurch gekennzeichnet,**
**dass** sie mehrere Kopien eines endogenen EPO-Gens jeweils in operativer Verknüpfung mit einem heterologen, in der humanen Zelle aktiven Promotor enthält und zur Produktion von mindestens 1000 ng EPO/10⁶ Zellen/24 h in der Lage ist, erhältlich durch ein Verfahren umfassend die Schritte:
(f) Amplifizieren des EPO-Gens und
(g) Gewinnen von EPO-produzierenden Zellen, die mehrere Kopien eines endogenen EPO-Gens jeweils in operativer Verknüpfung mit einer heterologen Expressionskontrollsequenz enthalten.

5. Humane Zelle nach Anspruch 4,
**dadurch gekennzeichnet,**
**dass** sie zur Produktion von 1000 - 25000 ng EPO/10⁶ Zellen/ 24 h in der Lage ist.

6. Humane Zelle nach einem der Ansprüche 1 bis 5,
**dadurch gekennzeichnet,**
**dass** sie eine immortalisierte Zelle ist.

7. Humane Zelle nach einem der Ansprüche 1 bis 6,
**dadurch gekennzeichnet,**
**dass** sie in serumfreiem Medium kultivierbar ist.

8. Humane Zelle nach einem der Ansprüche 1 bis 7,
**dadurch gekennzeichnet,**
**dass** sie aus einer HT 1080 Zelle, einer HeLa S3 Zelle, einer Namalwazelle oder einer davon abgeleiteten Zelle ausgewählt ist.

9. Humane Zelle nach einem der Ansprüche 1 bis 8,
**dadurch gekennzeichnet,**
**dass** das aktivierte endogene EPO-Gen unter Kontrolle eines CMV-Promotors ist.

10. Humane Zelle nach einem der Ansprüche 1 bis 9,
**dadurch gekennzeichnet,**
**dass** das EPO Gen eine Signaleptid-kodierende Sequenz aufweist, die von der natürlichen Signalpeptid-kodierenden Sequenz verschieden ist.

11. Humane Zelle nach Anspruch 10,
**dadurch gekennzeichnet,**
**dass** das EPO-Gen, das in operativer Verknüpfung mit der heterologen Expressionskontrollsequenz ist, eine Signalpeptid-kodierende Sequenz aufweist, die für eine im Bereich der 4 ersten Aminosäuren modifizierte Signalpeptidsequenz kodiert, die ausgewählt ist aus:
Met-X₁-X₂-X₃
worin X₁ Gly oder Ser ist, X₂ Ala, Val, Leu, Ile, Ser oder Pro ist, X₃ und Pro, Arg, Cys oder His ist, mit der Maßgabe, daß X₁-X₂-X₃ nicht die Sequenz Gly-Val-His ist.

12. Humane Zelle nach Anspruch 11,
**dadurch gekennzeichnet,**
**dass** die ersten 4 Aminosäuren ausgewählt sind aus:
(a) Met-Gly-Ala-His,
(b) Met-Ser-Ala-His,
(c) Met-Gly-Val-Pro oder
(d) Met-Ser-Val-His.

13. Humane Zelle nach Anspruch 12,
**dadurch gekennzeichnet,**
**dass** die Sequenz der ersten 4 Aminosäuren des Signalpeptids Met-Ser-Ala-His ist.

14. Verfahren zum Herstellen einer humanen EPO-produzierenden Zelle nach einem der Ansprüche 1 bis 13, umfassend die Schritte:
(a) Bereitstellen von humanen Ausgangszellen, die mindestens eine Kopie eines endogenen EPO-Gens enthalten, mit der Maßgabe, dass die humanen Ausgangszellen keine Keimbahnzellen sind.
(b) Transfizieren der Zellen mit einem DNA-Konstrukt umfassend:
(i) zwei flankierende DNA-Sequenzen, die homolog zu Bereichen des humanen EPO-Genlocus sind, um eine homologe Rekombination zu erlauben,
(ii) ein positives Selektionsmarkergen und
(iii) eine heterologe Expressionskontrollsequenz, die in der humanen Zelle aktiv ist, wobei die heterologe Expressionskontrollsequenz einen starken viralen Promotor umfasst und der Abstand zwischen dem heterologen Promotor und dem Translationsstart des EPO-Gens nicht größer als 1100 bp ist,
(c) Kultivieren der transfizierten Zellen unter Bedingungen, bei denen eine Selektion auf das Vorhandensein des positiven Selektionsmarkergens stattfindet,
(d) Analysieren der gemäß Schritt (c) selektierbaren Zellen und
(e) Identifizieren der EPO-produzierenden Zellen.

15. Verfahren nach Anspruch 14,
**dadurch gekennzeichnet,**
**dass** die homologen DNA-Sequenzen ausgewählt werden aus dem Bereich der 5'-untranslatierten Sequenzen, Exon 1 und Intron 1 des EPO-Gens.

16. Verfahren nach Anspruch 15,
**dadurch gekennzeichnet,**
**dass** man eine im Bereich von Exon 1 modifizierte Sequenz verwendet.

17. Verfahren nach den Ansprüchen 15 oder 16,
**dadurch gekennzeichnet,**
**dass** man das Neomycinphosphotransferasegen als Selektionsmarkergen verwendet.

18. Verfahren nach einem der Ansprüche 15 bis 17,
**dadurch gekennzeichnet,**
**dass** das DNA-Konstrukt weiterhin ein Amplifikationsgen umfasst.

19. Verfahren nach Anspruch 18,
**dadurch gekennzeichnet,**
**dass** man ein Dihydrofolatreduktasegen als Amplifikationsgen verwendet.

20. Verfahren nach Anspruch 19,
**dadurch gekennzeichnet,**
**dass** man das Gen einer Dihydrofolatreduktase Arginin-Mutante als Amplifikationsgen verwendet.

21. Verfahren nach einem der Ansprüche 16 bis 18, weiterhin umfassend die Schritte:
(f) Amplifizieren des EPO-Gens und
(g) Gewinnen von EPO-produzierenden Zellen, die mehrere Kopien eines endogenen EPO-Gens jeweils in operativer Verknüpfung mit einer heterologen Expressionskontrollsequenz enthalten.

22. Verfahren nach einem der Ansprüche 14 bis 21,
**dadurch gekennzeichnet,**
**dass** man einen CMV-Promotor/Enhancer als Expressionskontrollsequenz verwendet.

23. Verfahren nach einem der Ansprüche 14 bis 22,
**dadurch gekennzeichnet,**
**dass** man als DNA-Konstrukt das Plasmid p189 (DSM 11661) oder ein davon abgeleitetes Plasmid in linearisierter Form verwendet.

24. DNA-Konstrukt zur Aktivierung eines endogenen EPO-Gens in einer humanen Zelle, umfassend:
(i) zwei flankierende DNA-Sequenzen, die homolog zu Bereichen des humanen EPO-Genlocus ausgewählt aus 5'-untranslatierten Sequenzen, Exon 1 und Intron 1 sind, um eine homologe Rekombination zu erlauben, wobei im Bereich von Exon 1 eine modifizierte Sequenz vorliegt, kodierend für die Aminosäuren:
Met-X₁-X₂-X₃
worin X₁ Gly oder Ser ist, X₂ Ala, Val, Leu, Ile, Ser oder Pro ist, X₃ und Pro, Arg, Cys oder His ist, mit der Maßgabe, daß X₁-X₂-X₃ nicht die Sequenz Gly-Val-His ist,
(ii) ein positives Selektionsmarkergen,
(iii) eine heterologe Expressionskontrollsequenz, die in einer humanen Zelle aktiv ist, wobei die heterologe Expressionskontrollsequenz einen starken viralen Promotor umfasst und der Abstand zwischen dem heterologen Promotor und dem Translationsstart des EPO-Gens nicht größer als 1100 bp ist, und
(iv) gegebenenfalls ein Amplifikationsgen.

25. DNA-Konstrukt nach Anspruch 24,
**dadurch gekennzeichnet,**
**dass** die Aminosäuren ausgewählt sind aus
(a) Met-Gly-Ala-His,
(b) Met-Ser-Ala-His,
(c) Met-Gly-Val-Pro oder
(d) Met-Ser-Val-His.

26. Verwendung eines DNA-Konstrukts zur Aktivierung eines endogenen EPO-Gens in einer humanen Zelle zur Herstellung einer Zelle nach Anspruch 1, mit der Maßgabe, dass die humane Zelle keine Keimbahnzelle ist, wobei das Konstrukt umfasst:
(i) zwei flankierende DNA-Sequenzen, die homolog zu Bereichen des humanen EPO-Genlocus ausgewählt aus 5'-untranslatierten Sequenzen, Exon 1 und Intron 1 sind, um eine homologe Rekombination zu erlauben,
(ii) ein positives Selektionsmarkergen,
(iii) eine heterologe Expressionskontrollsequenz, die in einer humanen Zelle aktiv ist, wobei die heterologe Expressionskontrollsequenz einen starken viralen Promotor umfasst und der Abstand zwischen der heterologen Expressionskontrollsequenz und dem Translationstart des EPO-Gens nicht größer als 1100 bp ist, und
(iv) gegebenenfalls ein Amplifikationsgen.

27. Plasmid p189 (DSM 11661) oder ein davon abgeleitetes Plasmid.

28. Verfahren zur Herstellung von humanem EPO,
**dadurch gekennzeichnet,**
**dass** man eine humane Zelle nach einem der Ansprüche 1 bis 13 unter Bedingungen in einem geeigneten Medium kultiviert, bei denen eine Produktion von EPO erfolgt und das EPO aus dem Kulturmedium gewinnt.

29. Verfahren nach Anspruch 28,
**dadurch gekennzeichnet,**
**dass** man ein serumfreies Medium verwendet.

30. Verfahren nach einem der Ansprüche 28 oder 29,
**dadurch gekennzeichnet,**
**dass** man die Zellen in Suspension kultiviert.

31. Verfahren nach einem der Ansprüche 28 bis 30,
**dadurch gekennzeichnet,**
**dass** man die Kultivierung in einem Fermenter durchführt.

32. Verfahren nach Anspruch 31,
**dadurch gekennzeichnet,**
**dass** das Volumen des Fermenters 10 l - 50000 l beträgt.

33. Verfahren nach einem der Ansprüche 28 bis 32,
**dadurch gekennzeichnet,**
**dass** die Gewinnung des EPO aus dem Kulturmedium die Schritte umfasst:
(a) Leiten des Zellüberstands über ein Affinitätschromatographiemedium und Gewinnen der EPO enthaltenden Fraktionen,
(b) gegebenenfalls Leiten der EPO enthaltenden Fraktionen über ein hydrophobes-Interaktionschromatographiemedium und Gewinnen der EPO enthaltenden Fraktionen,
(c) Leiten der EPO enthaltenden Fraktionen über Hydroxyapatit und Gewinnen der EPO enthaltenden Fraktionen und
(d) Aufkonzentrierten oder/und Leiten über ein Reverse-Phase-HPLC-Medium.

34. Verfahren nach Anspruch 33,
**dadurch gekennzeichnet,**
**dass** man in Schritt (a) ein Blue-Sepharosemedium verwendet.

35. Verfahren nach einem der Ansprüche 33 oder 34,
**dadurch gekennzeichnet,**
**dass** man in Schritt (b) ein Butyl-Sepharosemedium verwendet.

36. Verfahren nach einem der Ansprüche 33 bis 35,
**dadurch gekennzeichnet,**
**dass** man das Aufkonzentrieren durch Ausschlußchromatographie durchführt.

37. Verfahren nach Anspruch 36,
**dadurch gekennzeichnet,**
**dass** man ein Medium mit einer Ausschlußgröße von 10 kD verwendet.

38. Verfahren nach einem der Ansprüche 28 bis 37,
**dadurch gekennzeichnet,**
**dass** man ein humanes EPO mit einer Reinheit von mindestens 90 % gewinnt.

39. Verfahren nach einem der Ansprüche 28 bis 38,
**dadurch gekennzeichnet,**
**dass** man ein humanes EPO mit einer spezifischen Aktivität in vivo (normozythämische Maus) von mindestens 100000 IU/mg gewinnt.

40. Verfahren nach Anspruch 39,
**dadurch gekennzeichnet,**
**dass** man ein humanes EPO mit einer spezifischen Aktivität in vivo (normozythämische Maus) von mindestens 175.000 IU/mg bis 450.000 IU/mg gewinnt.

41. Verfahren nach einem der Ansprüche 28 bis 40,
**dadurch gekennzeichnet,**
**dass** man ein humanes EPO mit einem Gehalt von weniger als 0,2 % N-Glycolneuraminsäure bezogen auf den Gehalt an N-Acetylneuraminsäure gewinnt.

42. Verfahren nach einem der Ansprüche 28 bis 41,
**dadurch gekennzeichnet,**
**dass** man ein humanes EPO mit α-2,3-verknüpften Sialinsäureresten gewinnt.

43. Verfahren nach einem der Ansprüche 28 bis 42,
**dadurch gekennzeichnet,**
**dass** man ein humanes EPO mit α-2,3- und α-2,6-verknüpften Sialinsäureresten gewinnt.

44. Verfahren nach einem der Ansprüche 28 bis 43,
**dadurch gekennzeichnet,**
**dass** man ein humanes EPO gewinnt, welches ein Polypeptid mit einer Länge von 165 Aminosäuren umfasst.

45. Verfahren nach einem der Ansprüche 28 bis 43,
**dadurch gekennzeichnet,**
**dass** man ein humanes EPO gewinnt, welches ein Polypeptid mit einer Länge von 166 Aminosäuren umfasst.

46. Verfahren nach einem der Ansprüche 28 bis 43,
**dadurch gekennzeichnet,**
**dass** man ein humanes EPO gewinnt, welches ein Gemisch aus Polypeptiden mit einer Länge von 165 und 166 Aminosäuren umfasst.

47. Isolierte DNA, die für ein humanes EPO mit einer im Bereich der ersten 4 Aminosäuren des Signalpeptids modifizierten Sequenz Met-Ser-Ala-His kodiert.

48. DNA nach Anspruch 47,
**dadurch gekennzeichnet,**
**dass** sie eine genomische DNA ist.

49. DNA nach Anspruch 47,
**dadurch gekennzeichnet,**
**dass** sie eine cDNA ist.

## Claims

1. Human cell,
**characterized in that**
it contains a copy of an endogenous EPO gene in operative linkage with a heterologous promoter that is active in the human cell and it is able to produce at least 200 ng EPO/10⁶ cells/24 h, the distance between the heterologous promoter and the translation start of the EPO gene being not more than 1100 bp, which can be obtained by a process comprising the steps:
(a) providing human starting cells which contain at least one copy of an endogenous EPO gene, provided that the human starting cells are not germ line cells,
(b) transfecting the cells with a DNA construct comprising:
(i) two flanking DNA sequences,which are homologous to regions of the human EPO gene locus in order to allow a homologous recombination,
(ii) a positive selection marker gene and
(iii) a heterologous expression control sequence which is active in the human cell, wherein the heterologous expression control sequence comprises a strong viral promoter and the distance between the heterologous promoter and the translation start of the EPO gene is not more than 1100 bp,
(c) culturing the transfected cells under conditions in which a selection takes place for the presence of the positive selection marker gene,
(d) analysing the cells that can be selected according to step (c) and
(e) identifying the EPO-producing cells.

2. Human cell according to claim 1,
**characterized in that**
it is able to produce 200 - 3000 ng EPO/10⁶ cells/24 h.

3. Human cell according to claim 1 or 2,
**characterized in that**
the cell is DSM ACC 2320.

4. Human cell obtainable by gene amplification from a cell according to one of the claims 1 to 3,
**characterized in that**
it contains several copies of an endogenous EPO gene each of which are in operative linkage with a heterologous promoter that is active in the human cell and is able to produce at least 1000 ng EPO/10⁶ cells/24 h, which can be obtained by a process comprising the steps:
(f) amplifying the EPO gene and
(g) isolating EPO-producing cells which contain several copies of an endogenous EPO gene each of which are in operative linkage with a heterologous expression control sequence.

5. Human cell according to claim 4,
**characterized in that**
it is able to produce 1000 - 25000 ng EPO/10⁶ cells/24 h.

6. Human cell according to one of the claims 1 to 5,
**characterized in that**
it is an immortalized cell.

7. Human cell according to one of the claims 1 to 6,
**characterized in that**
it can be cultured in serum-free medium.

8. Human cell according to one of the claims 1 to 7,
**characterized in that**
it is selected from a HT 1080 cell, a HeLa S3 cell, a Namalwa/cell or a cell derived therefrom.

9. Human cell according to one of the claims 1 to 8,
**characterized in that**
the activated endogenous EPO gene is under the control of a CMV promoter.

10. Human cell according to one of the claims 1 to 9,
**characterized in that**
the EPO gene has a signal peptide-coding sequence which is different from the natural signal peptide-coding sequence.

11. Human cell according to claim 10,
**characterized in that**
the EPO gene which is in operative linkage with the heterologous expression control sequence has a signal peptide-coding sequence which codes for a signal peptide sequence modified in the region of the first four amino acids which is selected from
Met-X₁-X₂-X₃
in which X₁ is Gly or Ser, X₂ is Ala, Val, Leu, Ile, Ser or Pro, X₃ is Pro, Arg, Cys or His provided that X₁-X₂-X₃ is not the sequence Gly-Val-His.

12. Human cell according to claim 11,
**characterized in that**
the first 4 amino acids are selected from:
(a) Met-Gly-Ala-His,
(b) Met-Ser-Ala-His,
(c) Met-Gly-Val-Pro or
(d) Met-Ser-Val-His.

13. Human cell according to claim 12,
**characterized in that**
the sequence of the first four amino acids of the signal peptide is Met-Ser-Ala-His.

14. Process for producing a human EPO-producing cell according to one of the claims 1 to 13, comprising the steps:
(a) providing human starting cells which contain at least one copy of an endogenous EPO gene, provided that the human starting cells are not germ line cells,
(b) transfecting the cells with a DNA construct comprising:
(i) two flanking DNA sequences which are homologous to regions of the human EPO gene locus in order to allow a homologous recombination,
(ii) a positive selection marker gene and
(iii) a heterologous expression control sequence which is active in the human cell, wherein the heterologous expression control sequence comprises a strong viral promoter and the distance between the heterologous promoter and the translation start of the EPO gene is not more than 1100 bp,
(c) culturing the transfected cells under conditions in which a selection takes place for the presence of the positive selection marker gene,
(d) analysing the cells that can be selected according to step (c) and
(e) identifying the EPO-producing cells.

15. Process according to claim 14,
**characterized in that**
the homologous DNA sequences are selected from the region of the 5' untranslated sequences, exon 1 and intron 1 of the EPO gene.

16. Process according to claim 15,
**characterized in that**
a sequence modified in the region of exon 1 is used.

17. Process according to one of the claims 15 or 16,
**characterized in that**
the neomycin phosphotransferase gene is used as the selection marker gene.

18. Process according to one of the claims 15 to 17,
**characterized in that**
the DNA construct additionally contains an amplification gene.

19. Process according to claim 18,
**characterized in that**
a dihydrofolate reductase gene is used as the amplification gene.

20. Process according to claim 19,
**characterized in that**
the gene of a dihydrofolate reductase arginine mutant is used as the amplification gene.

21. Process according to one of the claims 16 to 18, additionally comprising the steps:
(f) amplifying the EPO gene and
(g) obtaining EPO-producing cells which contain several copies of an endogenous EPO gene each of which is in operative linkage with a heterologous expression control sequence.

22. Process according to one of the claims 14 to 21,
**characterized in that**
a CMV promoter/enhancer is used as the expression control sequence.

23. Process according to one of the claims 14 to 22,
**characterized in that**
the plasmid p189 (DSM 11661) or a plasmid derived therefrom is used in a linearized form as the DNA construct.

24. DNA construct for activating an endogenous EPO gene in a human cell comprising:
(i) two flanking DNA sequences which are homologous to regions of the human EPO gene locus selected from 5' untranslated sequences, exon 1 and intron 1 in order to allow a homologous recombination, a modified sequence being present in the exon 1 region coding for the amino acids:
Met-X₁-X₂-X₃,
(ii) a positive selection marker gene,
(iii) a heterologous expression control sequence which is active in a human cell, wherein the heterologous expression control sequence comprises a strong viral promoter and the distance between the heterologous promoter and the translation start of the EPO gene is not more than 1100 bp, and
(iv) optionally an amplification gene.

25. DNA construct according to claim 24,
**characterized in that**
the amino acids are selected from
(a) Met-Gly-Ala-His,
(b) Met-Ser-Ala-His,
(c) Met-Gly-Val-Pro or
(d) Met-Ser-Val-His.

26. Use of a DNA construct to activate an endogenous EPO gene in a human cell for the production of a cell according to claim 1 provided that the human cell is not a germ line cell where the construct comprises:
(i) two flanking DNA sequences which are homologous to regions of the human EPO gene locus selected from 5' untranslated sequences, exon 1 and intron 1 in order to allow a homologous recombination,
(ii) a positive selection marker gene,
(iii) a heterologous expression control sequence which is active in a human cell, wherein the heterologous expression control sequence comprises a strong viral promoter and the distance between the heterologous expression control sequence and the translation start of the EPO gene is not more than 1100 bp, and
(iv) optionally an amplification gene.

27. Plasmid p189 (DSM 11661) or a plasmid derived therefrom.

28. Process for the production of human EPO,
**characterized in that**
a human cell according to one of the claims 1 to 13 is cultured in a suitable medium under conditions in which EPO is produced and EPO is isolated from the culture medium.

29. Process according to claim 28,
**characterized in that**
a serum-free medium is used.

30. Process according to one of the claims 28 or 29,
**characterized in that**
the cells are cultured in suspension.

31. Process according to one of the claims 28 to 30,
**characterized in that**
the culture is carried out in a fermenter.

32. Process according to claim 31,
**characterized in that**
the volume of the fermenter is 10 l- 50000 l.

33. Process according to one of the claims 28 to 32,
**characterized in that**
the isolation of EPO from the culture medium comprises the steps:
(a) passing the cell supernatant over an affinity chromatography medium and isolating the fractions containing EPO,
(b) optionally passing the fractions containing EPO over a hydrophobic interaction chromatography medium and isolating the fractions containing EPO,
(c) passing the fractions containing EPO over hydroxyapatite and isolating the fractions containing EPO and
(d) concentrating or/and passing over a reverse phase (RP) HPLC medium.

34. Process according to claim 33,
**characterized in that**
a blue Sepharose medium is used in step (a).

35. Process according to one of the claims 33 or 34,
**characterized in that**
a butyl Sepharose medium is used in step (b).

36. Process according to one of the claims 33 to 35,
**characterized in that**
the concentration is carried out by exclusion chromatography.

37. Process according to claim 36,
**characterized in that**
a medium with an exclusion size of 10 kD is used.

38. Process according to one of the claims 28 to 37,
**characterized in that**
a human EPO with a purity of at least 90 % is obtained.

39. Process according to one of the claims 28 to 38,
**characterized in that**
a human EPO having a specific activity in vivo (normocytic mouse) of at least 100,000 IU/mg is obtained.

40. Process according to claim 39,
**characterized in that**
a human EPO having a specific activity in vivo (normocytic mouse) of at least 175,000 IU/mg to 450,000 IU/mg is obtained.

41. Process according to one of the claims 28 to 40,
**characterized in that**
a human EPO with a content of less than 0.2 % N-glycol neuraminic acid based on the content of N-acetylneuraminic acid is obtained.

42. Process according to one of the claims 28 to 41,
**characterized in that**
a human EPO with α-2,3-linked sialic acid residues is obtained.

43. Process according to one of the claims 28 to 42,
**characterized in that**
a human EPO with α-2,3-linked and α-2,6-linked sialic acid residues is obtained.

44. Process according to one of the claims 28 to 43,
**characterized in that**
a human EPO is obtained which comprises a polypeptide of 165 amino acids in length.

45. Process according to one of the claims 28 to 43,
**characterized in that**
a human EPO is obtained which comprises a polypeptide of 166 amino acids in length.

46. Process according to one of the claims 28 to 43,
**characterized in that**
a human EPO is obtained which comprises a mixture of polypeptides of 165 and 166 amino acids in length.

47. Isolated DNA which codes for a human EPO with a modified sequence Met-Ser-Ala-His in the region of the first four amino acids of the signal peptide.

48. DNA according to claim 47,
**characterized in that**
it is a genomic DNA.

49. DNA according to claim 47,
**characterized in that**
it is a cDNA.

## Revendications

1. Cellule humaine
**caractérisée en ce qu'**elle contient une copie d'un gène EPO endogène en liaison opératoire avec un promoteur hétérologue actif dans la cellule humaine et qui est capable de produire au moins 200 ng d'EPO par 10⁶ cellules en 24 heures, la distance entre le promoteur hétérologue et le début de la traduction du gène EPO n'étant pas supérieure à 1 100 pb, susceptible d'être obtenue par un procédé qui comprend les étapes suivantes :
(a) fournir des cellules humaines de départ qui contiennent au moins une copie du gène EPO endogène, dans la mesure où les cellules humaines de départ ne sont pas des cellules du germen,
(b) transfecter les cellules par une structure d'ADN qui comprend
(I) deux séquences flanquantes d'ADN, homologues aux domaines du locus du gène EPO humain, pour permettre une recombinaison homologue,
(II) un gène marqueur de sélection positif,
(III) une séquence hétérologue de contrôle d'expression qui est active dans la cellule humaine, la séquence hétérologue de contrôle d'expression comprenant un fort promoteur viral et la distance entre le promoteur hétérologue et le début de la traduction du gène EPO n'étant pas supérieure à 1 100 pb,
(c) cultiver les cellules transfectées dans des conditions où une sélection a lieu en présence du gène marqueur positif de sélection,
(d) analyser les cellules qui peuvent être sélectionnées d'après l'étape (c) et
(e) identifier les cellules qui produisent l'EPO.

2. Cellule humaine selon la revendication 1, **caractérisée en ce qu'**elle est capable de produire de 200 à 3 000 ng d'EPO par 10⁶ cellules en 24 heures.

3. Cellule humaine selon la revendication 1 ou 2, **caractérisée en ce qu'**elle est la cellule DSM ACC 2 320.

4. Cellule humaine susceptible d'être obtenue par amplification de gène à partir d'une cellule selon l'une des revendications 1 à 3, **caractérisée en ce qu'**elle contient plusieurs copies d'un gène EPO endogène dont chacune est en liaison opératoire avec un promoteur hétérologue actif dans la cellule humaine et qui est capable de produire au moins 1 000 ng d'EPO par 10⁶ cellules en 24 heures, susceptible d'être obtenue par un procédé qui comprend les étapes suivantes :
(f) amplifier le gène EPO et
(g) obtenir des cellules qui produisent de l'EPO et qui contiennent plusieurs copies d'un gène EPO endogène dont chacune est en liaison opératoire avec une séquence hétérologue de contrôle d'expression.

5. Cellule humaine selon la revendication 4, **caractérisée en ce qu'**elle est en mesure de produire entre 1 000 et 25 000 ng d'EPO par 10⁶ cellules en 24 heures.

6. Cellule humaine selon l'une des revendications 1 à 5, **caractérisée en ce qu'**elle est une cellule immortalisée.

7. Cellule humaine selon l'une des revendications 1 à 6, **caractérisée en ce qu'**elle peut être cultivée dans un milieu sans sérum.

8. Cellule humaine selon l'une des revendications 1 à 7, **caractérisée en ce qu'**elle est sélectionnée parmi une cellule HT 1 080, une cellule HeLa S3, une cellule Namalwa ou une cellule qui en est dérivée.

9. Cellule humaine selon l'une des revendications 1 à 8, **caractérisée en ce que** le gène EPO endogène activé est sous contrôle d'un promoteur CMV.

10. Cellule humaine selon l'une des revendications 1 à 9, **caractérisée en ce que** le gène EPO comporte une séquence qui code pour les peptides signal et qui est différente de la séquence naturelle qui code pour les peptides signal.

11. Cellule humaine selon la revendication 10, **caractérisée en ce que** le gène EPO qui est en liaison opératoire avec la séquence hétérologue de contrôle d'expression comporte une séquence codante pour le peptide signal qui code pour une séquence de peptides signal, modifiée dans le domaine des 4 premiers acides aminés, sélectionnée parmi
Met-X₁-X₂-X₃
où X₁ est Gly ou Ser, X₂ est Ala, Val, Leu, Ile, Ser ou Pro, X₃ est Arg, Cys ou His dans la mesure où X₁-X₂-X₃ n'est pas la séquence Gly-Val-His.

12. Cellule humaine selon la revendication 11, **caractérisée en ce que** les quatre premiers acides aminés sont sélectionnés parmi
(a) Met-Gly-Ala-His
(b) Met-Ser-Ala-His
(c) Met-Gly-Val-Pro ou
(d) Met-Ser-Val-His.

13. Cellule humaine selon la revendication 12, **caractérisée en ce que** la séquence des quatre premiers acides aminés du peptide signal est Met-Ser-Ala-His.

14. Procédé pour obtenir une cellule humaine qui produit l'EPO d'après l'une des revendications 1 à 13, comprenant les étapes suivantes
(a) fournir des cellules humaines de départ qui contiennent au moins une copie du gène EPO endogène, dans la mesure où les cellules humaines de départ ne sont pas des cellules du germen,
(b) transfecter les cellules par une structure d'ADN qui comprend
(I) deux séquences flanquantes d'ADN, homologues aux domaines du locus du gène EPO humain, pour permettre une recombinaison homologue,
(II) un gène marqueur de sélection positif,
(III) une séquence hétérologue de contrôle d'expression qui est active dans la cellule humaine, la séquence hétérologue de contrôle d'expression comprenant un fort promoteur viral et la distance entre le promoteur hétérologue et le début de la traduction du gène EPO n'étant pas supérieure à 1 100 pb,
(c) cultiver les cellules transfectées dans des conditions où une sélection a lieu en présence du gène marqueur positif de sélection,
(d) analyser les cellules qui peuvent être sélectionnées d'après l'étape (c) et
(e) identifier les cellules qui produisent l'EPO.

15. Procédé selon la revendication 14, **caractérisé en ce que** les séquences d'ADN homologues sont sélectionnées dans le domaine des séquences non traduites 5', exon 1 et intron 1 du gène EPO.

16. Procédé selon la revendication 15, **caractérisé en ce qu'**on utilise une séquence modifiée dans le domaine d'exon 1.

17. Procédé selon les revendications 15 ou 16, **caractérisé en ce que** le gène marqueur de sélection utilisé est le gène de la néomycine phosphotransférase.

18. Procédé selon l'une des revendications 15 à 17, **caractérisé en ce que** la structure d'ADN comprend en outre un gène d'amplification.

19. Procédé selon la revendication 18, **caractérisé en ce que** le gène d'amplification utilisé est un gène de la dihydrofolate réductase.

20. Procédé selon la revendication 19, **caractérisé en ce que** le gène d'amplification utilisé est le gène d'un mutant arginine de la dyhydrofolate réductase.

21. Procédé selon l'une des revendications 16 à 18, qui comprend en outre les étapes
(f) amplifier le gène EPO et
(g) obtenir des cellules produisant l'EPO et qui contiennent plusieurs copies d'un gène EPO endogène dont chacun est en liaison opératoire avec une séquence hétérologue de contrôle d'expression.

22. Procédé selon l'une des revendications 14 à 21, **caractérisé en ce que** la séquence de contrôle d'expression utilisée est un activateur ou un promoteur de CMV.

23. Procédé selon l'une des revendications 14 à 22, **caractérisé en ce que** la structure d'ADN utilisée est le plasmide p 189 (DSM 11 661) ou un plasmide qui en est dérivé sous forme linéarisée.

24. Structure d'ADN destinée à activer un gène EPO endogène dans une cellule humaine, comprenant
(I) deux séquences flanquantes d'ADN, homologues à des domaines du locus du gène EPO humain et sélectionnées parmi les séquences non traduites 5', exon 1 et intron 1, afin de permettre une recombinaison homologue, il y a dans le domaine d'exon 1 une séquence modifiée qui code pour les acides aminés
Met-X₁-X₂-X₃
où X₁ est Gly ou Ser, X₂ est Ala, Val, Leu, Ile, Ser ou Pro, X₃ est Pro, Arg, Cys ou His dans la mesure où X₁-X₂-X₃ n'est pas la séquence Gly-Val-His.
(II) un gène marqueur de sélection positif,
(III) une séquence hétérologue de contrôle d'expression qui est active dans la cellule humaine, la séquence hétérologue de contrôle d'expression comprenant un fort promoteur viral et la distance entre le promoteur hétérologue et le début de la traduction du gène EPO n'étant pas supérieure à 1 100 pb et
(IV) éventuellement un gène d'amplification.

25. Structure d'ADN selon la revendication 24, **caractérisée en ce que** les acides aminés sont sélectionnés parmi
(a) Met-Gly-Ala-His
(b) Met-Ser-Ala-His
(c) Met-Gly-Val-Pro ou
(d) Met-Ser-Val-His.

26. Utilisation d'une structure d'ADN pour activer un gène EPO endogène dans une cellule humaine pour obtenir une cellule d'après la revendication 1, dans la mesure où la cellule humaine de départ n'est pas une cellule de germen, la structure comprenant
(I) deux séquences flanquantes d'ADN, homologues aux domaines du locus du gène EPO humain et sélectionnées parmi les séquences non traduites 5', exon 1 et intron 1, afin de permettre une recombinaison homologue,
(II) un gène marqueur de sélection positif,
(III) une séquence hétérologue de contrôle d'expression qui est active dans la cellule humaine, la séquence hétérologue de contrôle d'expression comprenant un fort promoteur viral et la distance entre le promoteur hétérologue et le début de la traduction du gène EPO n'étant pas supérieure à 1 100 pb et
(IV) éventuellement un gène d'amplification.

27. Plasmide p189 (DSM 11 661) ou un plasmide qui en est dérivé.

28. Procédé pour obtenir des EPO humains, **caractérisé en ce qu'**on cultive une cellule humaine d'après l'une des revendications 1 à 13 dans un milieu adapté, dans des conditions où il y a production d'EPO et où on obtient l'EPO à partir du milieu de culture.

29. Procédé selon la revendication 28, **caractérisé en ce qu'**on utilise un milieu sans sérum.

30. Procédé selon l'une des revendications 28 ou 29, **caractérisé en ce qu'**on cultive les cellules en suspension.

31. Procédé selon l'une des revendications 28 à 30, **caractérisé en ce que** la culture a lieu dans un fermenteur.

32. Procédé selon la revendication 31, **caractérisé en ce que** le volume du fermenteur est compris entre 10 l et 50 000 l.

33. Procédé selon l'une des revendications 28 à 32, **caractérisé en ce que** l'obtention de l'EPO à partir du milieu de culture comprend les étapes suivantes :
(a) passer le surnageant de cellule sur une phase pour chromatographie d'affinité et obtenir les fractions contenant l'EPO,
(b) éventuellement passer les fractions contenant l'EPO sur une phase pour chromatographie d'interaction hydrophobe et obtenir les fractions contenant l'EPO,
(c) passer les fractions contenant l'EPO par de l'hydroxyapatite et obtenir les fractions contenant l'EPO,
(d) concentrer et/ou passer sur une phase inversée pour CHPL.

34. Procédé selon la revendication 33, **caractérisé en ce qu'**on utilise une phase Blue-Sépharose à l'étape (a).

35. Procédé selon l'une des revendications 33 ou 34, **caractérisé en ce qu'**on utilise une phase de butyle-Sépharose à l'étape (b).

36. Procédé selon l'une des revendications 33 à 35, **caractérisé en ce que** la concentration a lieu par chromatographie d'exclusion.

37. Procédé selon la revendication 36, **caractérisé en ce qu'**on utilise une phase dont le niveau d'exclusion est de 10 kD.

38. Procédé selon l'une des revendications 28 à 37, **caractérisé en ce qu'**on obtient un EPO humain dont la pureté est d'au moins 90 %.

39. Procédé selon l'une des revendications 28 à 38, **caractérisé en ce qu'**on obtient un EPO humain dont l'activité spécifique in vivo (souris normocythémique) est d'au moins 100 000 UI/mg.

40. Procédé selon la revendication 39, **caractérisé en ce qu'**on obtient un EPO humain dont l'activité spécifique in vivo (souris normocythémique) est d'au moins 175 000 UI/mg à 450 000 UI/mg.

41. Procédé selon l'une des revendications 28 à 40, **caractérisé en ce qu'**on obtient un EPO humain dont la teneur en acide N-glycol-neuraminique est inférieure à 0,2 % sur la base de la teneur en acide N-acétyl-neuraminique.

42. Procédé selon l'une des revendications 28 à 41, **caractérisé en ce qu'**on obtient un EPO humain à radicaux acide sialique liés en α-2,3.

43. Procédé selon l'une des revendications 28 à 42, **caractérisé en ce qu'**on obtient un EPO humain à radicaux acide sialique liés en α-2,3 et α-2,6.

44. Procédé selon l'une des revendications 28 à 43, **caractérisé en ce qu'**on obtient un EPO humain qui comprend un polypeptide d'une longueur de 165 acides aminés.

45. Procédé selon l'une des revendications 28 à 43, **caractérisé en ce qu'**on obtient un EPO humain qui comprend un polypeptide d'une longueur de 166 acides aminés.

46. Procédé selon l'une des revendications 28 à 43, **caractérisé en ce qu'**on obtient un EPO humain qui comprend un mélange de polypeptides d'une longueur de 165 et de 166 acides aminés.

47. ADN isolé qui code pour un EPO humain par une séquence Met-Ser-Ala-His modifiée dans la zone des 4 premiers acides aminés du peptide signal.

48. ADN selon la revendication 47, **caractérisé en ce qu'**il est un ADN génomique.

49. ADN selon la revendication 47, **caractérisé en ce qu'**il est un ADN-c.


### SEQUENZPROTOKOLL

(1) ALLGEMEINE ANGABEN:
   (i) ANMELDER:
      (A) NAME: Boehringer Mannheim GmbH
      (B) STRASSE: Sandhofer Str. 112-132
      (C) ORT: Mannheim
      (E) LAND: Deutschland
      (F) POSTLEITZAHL: 68305
   (ii) BEZEICHNUNG DER ERFINDUNG: Herstellung von Erythropoietin durch endogene Genaktivierung
   (iii) ANZAHL DER SEQUENZEN: 17
   (iv) COMPUTER-LESBARE FASSUNG:
      (A) DATENTRAGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 32 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 1:
      CGCGGCGGAT CCCAGGGAGC TGGGTTGACC GG 32
(2) ANGABEN ZU SEQ ID NO: 2:
   (i) SEQUENZKENNZEICHEN:
      (A) LANGE: 38 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 2:
      GGCCGCGAAT TCTCCGCGCC TGGCCGGGGT CCCTCAGC 38
(2) ANGABEN ZU SEQ ID NO: 3:
   (i) SEQUENZKENNZEICHEN:
      (A) LANGE: 36 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 3:
      CGCGGCGGAT CCTCTCCTCC CTCCCAAGCT GCAATC 36
(2) ANGABEN ZU SEQ ID NO: 4:
   (i) SEQUENZKENNZEICHEN:
      (A) LÄNGE: 36 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 4:
      CGCCGCGAAT TCTAGAACAG ATAGCCAGGC TGAGAG 36
(2) ANGABEN ZU SEQ ID NO: 5:
   (i) SEQUENZKENNZEICHEN:
      (A) LÄNGE: 24 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 5:
      TCACCCGGCG CGCCCCAGGT CGCT 24
(2) ANGABEN ZU SEQ ID NO: 6:
   (i) SEQUENZKENNZEICHEN:
      (A) LÄNGE: 61 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 6:
   ATGCTCGAGC GGCCGCCAGT GTGATGGATA TCTGCAGAGC TCAGCTTGGC CGCGAATTCT 60
   A 61
(2) ANGABEN ZU SEQ ID NO: 7:
   (i) SEQUENZKENNZEICHEN:
      (A) LÄNGE: 78 Basenpaare
      (B) ART: Nucleotid
      (C) STRANGFORM: Einzelstrang
      (D) TOPOLOGIE: linear
   (ix) MERKMAL:
      (A) NAME/SCHLÜSSEL: CDS
      (B) LAGE:49..60
   (xi) SEQUENZBESCHREIBUNG: SEQ ID NO: 7:
   TCACCCGGCG CGCCCCAGGT CGCTGAGGGA CCCCGGCCAG GCGCGGAG ATG GGG GCC        57
                                                        Met Gly Ala
                                                         1
   CAC GGTGAGTACT CGCGGGCT                                                  78
   His
(2) ANGABEN ZU SEQ ID NO: 8:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKÜLS: Protein
   (Xi) SEQUENZBESCHREIBUNG: SEQ ID NO: 8:
   Met Gly Ala His
    1
(2) ANGABEN ZU SEQ ID NO: 9:
   (i) SEQUENZKENNZEICHEN:
      (A) LÄNGE: 78 Basenpaare
      (B) ART: Nucleotid
      (C) STRANGFORM: Einzelstrang
      (D) TOPOLOGIE: linear
   (ix) MERKMAL:
      (A) NAME/SCHLÜSSEL: CDS
      (B) LAGE:49..60
   (xi) SEQUENZBESCHREIBUNG: SEQ ID NO: 9:
   TCACCCGGCG CGCCCCAGGT CGCTGAGGGA CCCCGGCCAG GCGCGGAG ATG AGC GCC        57
                                                        Met Ser Ala
                                                         5
   CAC GGTGAGTACT CGCGGGCT                                                  78
   His
(2) ANGABEN ZU SEQ ID NO: 10:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 10:
   Met Ser Ala His
    1
(2) ANGABEN ZU SEQ ID NO: 11:
   (i) SEQUENZKENNZEICHEN:
      (A) LÄNGE: 78 Basenpaare
      (B) ART: Nucleotid
      (C) STRANGFORM: Einzelstrang
      (D) TOPOLOGIE: linear
   (ix) MERKMAL:
      (A) NAME/SCHLÜSSEL: CDS
      (B) LAGE:49..60
   (xi) SEQUENZBESCHREIBUNG: SEQ ID NO: 11:
   TCACCCGGCG CGCCCCAGGT CGCTGAGGGA CCCCGGCCAG GCGCGGAG ATG GGG GTG     57
                                                        Met Gly Val
                                                         5
   CCC GGTGAGTACT CGCGGGCT                                              78
   Pro
(2) ANGABEN ZU SEQ ID NO: 12:
   (i) SEQUENZKENNZEICHEN:
      (A) LÄNGE: 4 Aminosäuren
      (B) ART: Aminosäure
      (D) TOPOLOGIE: linear
   (ii) ART DES MOLEKÜLS: Protein
   (Xi) SEQUENZBESCHREIBUNG: SEQ ID NO: 12:
   Met Gly Val Pro
    1
(2) ANGABEN ZU SEQ ID NO: 13:
   (i) SEQUENZKENNZEICHEN:
      (A) LÄNGE: 78 Basenpaare
      (B) ART: Nucleotid
      (C) STRANGFORM: Einzelstrang
      (D) TOPOLOGIE: linear
   (ix) MERKMAL:
      (A) NAHE/SCHLÜSSEL: CDS
      (B) LAGE:49..60
   (xi) SEQUENZBESCHREIBUNG: SEQ ID NO: 13:
   TCACCCGGCG CGCCCCAGGT CGCTGAGGGA CCCCGGCCAG GCGCGGAG ATG AGC GTG 57
   Met Ser Val
   5
   CAC GGTGAGTACT CGCGGGCT 78
   His
(2) ANGABEN ZU SEQ ID NO: 14:
   (i) SEQUENZKENNZEICHEN:
      (A) LANGE: 4 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 14:
   Met Ser Val His
   1
(2) ANGABEN ZU SEQ ID NO: 15:
   (i) SEQUENZKENNZEICHEN:
      (A) LÄNGE: 59 Basenpaare
      (B) ART: Nucleotid
      (C) STRANGFORM: Einzelstrang
      (D) TOPOLOGIE: linear
   (Xi) SEQUENZBESCHREIBUNG: SEQ ID NO: 15:
      GGACATTCTA GAACAGATAT CCAGGCTGAG CGTCAGGCGG GGAGGGAGAA GGGTGGCTG 59
(2) ANGABEN ZU SEQ ID NO: 16:
   (i) SEQUENZKENNZEICHEN:
      (A) LÄNGE: 48 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 16:
      GTGATGGATA TCTCTAGAAC AGATAGCCAG GCTGAGAGTC AGGCGGGG 48
(2) ANGABEN ZU SEQ ID NO: 17:
   (i) SEQUENZKENNZEICHEN:
      (A) LÄNGE: 39 Basenpaare
      (B) ART: Nucleotid
      (C) STRANGFORM: Einzelstrang
      (D) TOPOLOGIE: linear
   (xi) SEQUENZBESCHREIBUNG: SEQ ID NO: 17:
      ATGGATATCA TCGATTCTAG AACAGATAGC CAGGCTGAG 39